# EUROPEAN PATENT APPLICATION

(11) **EP 3 396 378 A1**
(43) Date of publication of application: **31.10.2018**
(21) Application number: 17167789.1
(22) Date of filing: 24.04.2017
(51) Int. Cl.: G01N 33/569, C12Q 1/68

(54) **A METHOD FOR DETERMINING MYELOID NATURAL KILLER (NK)-CELLS AND USE THEREOF**

(71) Applicant: Max-Planck-Gesellschaft zur Förderung der Wissenschaften e.V., 80539 München (DE)
(72) Inventor: BRÜNING, Jens, 50933 Köln (DE); THEURICH, Sebastian, 50937 Köln (DE)
(74) Representative: Arth, Hans-Lothar

(57) **Abstract**

The present invention relates to ex-vivo methods for determining myeloid NK-cells, methods for diagnosis of a disease associated with and/or caused by myeloid NK-cells as well as depletion of myeloid NK-cells for use in treating. The present is also related to methods for determining whether a candidate agent reduces a myeloid NK-cell population.

## Description

The present invention relates to ex-vivo methods for determining myeloid NK-cells, methods for diagnosis of a disease associated with and/or caused by myeloid NK-cells as well as depletion of myeloid NK-cells for use in treating. The present is also related to methods for determining whether a candidate agent reduces a myeloid NK- cell population.

### Background of the invention

Immune cells reside in organs under physiological conditions and serve as important regulators of tissue homeostasis, overnutrition and a sedentary lifestyle imbalance energy and tissue homeostasis and finally propagate a state termed metaflammation.

Metaflammation represents a chronic, systemic low-grade immune activation that contributes to the development of the metabolic syndrome, i.e. obesity, type 2 diabetes, dyslipidemia and atherosclerosis. Whereas macrophages and their pro-inflammatory polarization have been initially described as key factors in the development of metaflammation, during the recent years the number of other immune cell populations identified to contribute to the development and maintenance of obesity-associated metaflammation has constantly increased. However, in contrast to innate lymphoid cells (ILC), natural killer T (NKT) cells and B cells, which have been more extensively characterized in obesity-associated inflammation, the specific role of classic natural killer (NK)-cells in metaflammation remains ill defined. This is surprising as in lean mice, NK-cells are the major immune cell population residing in white adipose tissue. In humans, most studies on obesity-associated changes in circulating NK-cells report on their increased activation status with less cytotoxic capacity. In mice, obesity promotes the maturation of NK-cells.

NK-cells contribute to the development of obesity-associated insulin resistance. The inventors could demonstrate that in mice obesity promotes a selective increase of myeloid, CD11b^{hi}-expressing NK-cells. Unlike classical NK-cells, they express myeloid marker genes including the IL-6 receptor alpha (IL6Ra) and Csf-1 receptor (Csf1r). Also in humans, myeloid NK-cells increase in obesity, decrease upon weight loss and share the myeloid gene signature of murine CD11b^{hi} NK-cells. Selective ablation of myeloid NK-cells in mice prevents obesity and insulin resistance. Conditional inactivation of IL6Ra or Stat3 limits formation of myeloid NK-cells in obesity, protects from obesity, insulin resistance and obesity-associated inflammation. Collectively, obesity-associated pathologies depend on IL-6/Stat3-dependent formation of a distinct myeloid NK-cell subset, which may provide a novel target for obesity and diabetes treatment as well as in diseases, in which chronic inflammation or metaflammation promotes formation of these myeloid NK-cell subsets.

### Brief Summary of the Invention

The objective of the present invention is to provide specific targets for the treatment and monitoring of diseases, such as obesity, diabetes and diseases in which metaflammation is involved. This goal is achieved by identification of a distinct subset NK-cell population interfering with progression of obesity and insulin resistance.

The present invention describes natural killer (NK) cells that show in addition to the expression of certain NK-cell-defining genes also expression of higher CD11b levels than conventional NK-cells and several genes that are usually annotated to myeloid immune cells. This gene list includes Csf1r, IL6Ra and several other myeloid marker genes, as listed in the following. The cells, newly described herein, are hereafter named as CD11b^{hi} or myeloid NK-cells. Transcriptome analyses of FACS-sorted, single NK-cells and subsequent next-generation RNA-sequencing of each individual single cell strongly suggest that CD11b^{hi} NK-cells develop from conventional NK-cells. To morphologically distinguish CD11bhi NK-cells from conventional NK-cells, FACS analysis and microscopic studies were performed, which showed that CD11b^{hi} NK-cells are larger and are also more granulated. This phenotype can reflect a higher activation status of these cells and also a possible myeloid-like differentiation.

Thus, in the present invention, it was surprisingly possible to identify both in obese mice and humans a unique, previously undefined NK-cell subpopulation, i.e. CD11b^{hi} NK-cells, which is characterized by the additional expression of myeloid genes, high CD11b expression and morphological features of large, granulated cells that accumulate in adipose tissue and the circulation of obese mice. Specific ablation of this cell population in mice - even in the absence of altered overall NK-cell numbers - protects from the development of obesity and insulin resistance upon high-fat diet feeding. Moreover, interleukin-6 (IL-6)-/signal-transducer-and-activator-of-transcription-3 (Stat3)-signaling was identified as a critical determinant for formation and adipose tissue recruitment of this specific NK-cell subpopulation in vivo and in the establishment of obesity and obesity-associated inflammation and insulin resistance.

The invention refers particularly to an ex-vivo method for determining myeloid NK-cells, wherein the method comprises provision of a sample containing myeloid NK-cells; labeling the myeloid NK-cells of the sample and detection of the myeloid NK-cells. Thereby the myeloid NK-cells may be separated from the rest of the sample and/or may be quantified. It is thereby preferred to label at least one distinct marker of the myeloid NK-cells, but it is also possible to apply the myeloid/NK-cell gene set that was found in these cells or a combination of a conventional NK-cell marker together with a myeloid marker.

The invention further provides a method for treatment of diseases involving an increase or activation of myeloid NK-cells, such as obesity, insulin resistance, diabetes, autoimmune diseases, cancer (particularly obesity-associated cancer), chronic infections or inflammation by depletion or inhibition of myeloid NK-cells. The invention refers further to a pharmaceutical composition comprising a compound depleting or inhibiting myeloid NK-cells for the treatment of these diseases. Depletion or inhibition of myeloid NK-cells can be done by inhibition of the maturation of this subpopulation. One possibility is the inhibition of one specific signaling pathway of these cells, like IL-6/Stat3-dependent signaling, e.g. by using inhibitors of Stat3 or IL-6 antagonists. Alternatively, one can use (bispecific) antibodies binding myeloid NK-cells. Using these antibodies one can deplete myeloid NK-cells by affinity chromatography.

The invention relates also to a method for diagnosis of a disease associated with and/or caused by myeloid NK-cells which comprises labeling of myeloid NK-cells of a sample and detecting the marked myeloid NK-cells. It is preferred that thereby the labeled myeloid NK-cells are quantified and subsequently compared with standard value. The disease associated with and/or caused by myeloid NK-cells is preferably selected from a group consisting of or comprising obesity, insulin resistance, diabetes, autoimmune diseases, (obesity-associated) cancer, chronic infections and inflammation.

Another embodiment of the present invention refers to a method for determining whether a candidate agent reduces a myeloid NK-cell population which comprises contacting a sample containing the myeloid NK-cell population with the candidate agent and subsequently labeling the myeloid NK-cell population of the sample. Thereafter the myeloid NK-cell population is detected and it is determined if the candidate agent reduces the myeloid NK-cell population. Alternatively, it is detected if the candidate agent inhibits activity of myeloid NK-cells. This inventive method may include determination if the candidate agent inhibits the activity of at least one protein of a group containing IL6Ra, Csf1r, gp130, JAK1/2, Spi1,STAT3, Pla2g7, Fos, Csf1r, Cd93, Mpeg1, Cybb, Ctss, Spi1, Il6ra, Cd74, Plbd1, Cd14, Clec10a, Il1rn, Sirpa, Pid1, Ptafr, Ly86, Grn, Tgfbi, Ctsh, C1qc, C1qb, Mrc1, Lrp1, Csf2ra, Ncf1, Cxcl9, Cd302, Cd300lb, Nfam1, Trem2, Emilin2, App, Sdc3, Ifi30, Csf2rb, Igsf6, Marcks, Ctsb, Cst3, Hp, Cfp, Lgals3, Cd300ld, Ifngr2, Rasgrp4, Scpep1, Fgd4, Basp1, Ctsz, Slc11a1, Clec12a, Gm2a, Adap2, Msrb1, Trib1, Msr1, Il1b, Klf4, Hck, Acer3, Plekho1, Mafb, Ciita, Axl, Adam15, Mef2c, Cebpb, Cebpa, Dusp1, Ext1, C1qa, Unc93b1, Naaa, Tmem86a, Lst1, Atf3, Ptpro, Nav1, Pld4, Tlr1, Pou2f2, Lacc1, Themis2, Ccdc109b, Ms4a7, and Rassf4, wherein IL6Ra, Csf1r, gp130, JAK1/2, Spi1 and STAT3 are prefered.

Further advantageous embodiments, aspects and details of the invention are evident from the depending claims, the detailed description, the examples and the figures.

### Detailed Description of the Invention

Hematopoietic Stem Cells (HSCs) are able to differentiate into cells of two primary lineages, lymphoid and myeloid. Cells of the myeloid lineage develop during the process of myelopoiesis and include Granulocytes, Monocytes, Megakaryocytes, and Dendritic Cells. Circulating Erythrocytes and Platelets also develop from myeloid progenitor cells. Lymphoid cells are a group of immune cells derived from common lymphoid progenitor (CLP) and belong to the lymphoid lineage. These cells are defined by absence of antigen specific B or T cell receptor because of the lack of recombination activating gene (RAG). Usually, innate lymphoid cells do not express myeloid or dendritic cell markers. NK-cells are cytotoxic lymphocytes and important player of the innate immune system. NK-cells are defined as large granular lymphocytes (LGL) and constitute kind of cells differentiated from the common lymphoid progenitor-generating B and T lymphocytes, thus they belong to the group of innate lymphoid cells. In humans, NK-cells usually are defined as CD3⁻CD56⁺ lymphocyte. NK-cells have cytotoxic granules containing perforin and various granzymes, leading to the perforation of target cells and subsequent apoptotic death. induced by the permeated granzymes (Liebermann, 2003; Voskoboinik et al., 2006). In addition, NK-cells express and secrete ligands (FasL, TRAIL and TNF) of tumor necrosis factor (TNF) receptor superfamily (TNFRSF) members, such as Fas/CD95, TRAIL receptors and TNFR1. These Ligands contributes to NK cytotoxicity against tumor cells. Further, NK-cells may secrete a variety of cytokines and chemokines, including interferon-γ (IFN-γ), TNF, and GM-CSF (granulocyte-macrophage colony stimulating factor), MIP-1α (macrophage inflammatory protein-1α). Cytokine secretion is triggered by detecting major histocompatibility complex (MHC) presented on infected cell surfaces.

Tissue resident immune cells control tissue homeostasis and metabolism in lean individuals, but, on the other hand, give rise to low-grade tissue inflammation, i.e. metaflammation, in obesity. The cellular network of metaflammation is complex and so far, only incompletely understood. Studies in obese mouse models have only very recently revealed the general importance of classical NK-cells for metaflammation and glucose metabolism. Despite this progress, it remained enigmatic whether conventional NK-cells contribute to the development of metaflammation or whether a distinct subset or a specialized NK-cell population interferes with progression of obesity and insulin resistance. The present invention defines a unique and novel NK-cell population characterized by high CD11b expression, which is induced in obese mice and humans. The examples demonstrate that this distinct subpopulation of CD11b^{hi} NK-cells are specific and important mediators of metaflammation and insulin resistance as their specific ablation reduces inflammation and decreases body weight accompanied by improved insulin sensitivity even in the absence of alterations in the overall number of NK-cells.

That newly identified subpopulation of NK-cells express a large number of myeloid marker genes and share transcription profiles with myeloid cells including dendritic cells, monocytes, macrophages and granulocytes. In line with this, the CD11b^{hi} NK-cells exhibit morphologic features of myeloid cells. However, NK-cell features remain dominant as the majority of genes - including genes characteristic for NK-cells - are still shared in both, mature and CD11b^{hi} NK-cells marking these cells as of the NK-cell lineage. Interestingly, based on transcriptional analyses of enriched upstream regulatory pathways involved in the development of the myeloid NK-cells and pre-mNK revealed that insulin and IL-6 signaling might be shared regulators of both cell types. It is proposed that myeloid NK-cells represent intermediate developmental stages that accumulate during NK differentiation due to the specific microenvironment created by metaflammation. Myeloid NK-cells are defined by the classical NK-cell immunophenotype of cell surface markers, and additional expression of markers of myeloid lineage cells, i.e. IL6Ra and Csf1r. The classical NK-cell surface marker in humans are CD45+CD3-CD56+, and NKp46+ wherein in mice they are CD45+CD3-NK1.1+, and NKp46+.

Nevertheless, the current invention unequivocally defines IL-6/Stat3-dependent signaling as a pre-requisite for the formation of myeloid, CD11b^{hi} NK-cells in obese mice. Moreover, and consistent with the myeloid features of CD11b^{hi} NK-cells, IL-6 signaling is a critical regulator of myeloid cells and for subgroups of activated Band T-cells. Classic IL-6-signaling is induced by binding of IL-6 to its receptor, IL6Ra, expressed on specific target cells followed by oligomerization with the ubiquitously expressed common cytokine signaling chain gp130. On the other hand, trans-signaling can occur in cells lacking the IL6Ra when IL-6 binds to soluble IL6Ra which then dimerizes with gp130. The presented results in transgenic mice either lacking IL6Ra or Stat3 specifically in NK-cells show that both components in NK-cells are mediating the formation of the myeloid NK-cells. This further confirms the NK-cell origin of these cells and indicates that the IL-6-dependent formation of the myeloid NK-cells occurs through classical rather than trans-signaling. In line with this conclusion, Kraakmann et al. (Cell 477 Metabolism. 21, 403-416 (2015)) recently showed that blocking of IL-6 trans-signaling inhibits macrophage accumulation into adipose tissue but does not improve insulin sensitivity or weight gain. Importantly, these authors propose that cell-type specific IL-6 blockade rather than global inhibition of IL-6 signaling should be considered for therapeutic interventions, which further underscores the impact of our findings.

Taken together, the examples of the present application demonstrate that obesity-associated pathologies critically depend on IL-6/Stat3-dependent formation of a unique myeloid lineage NK-cell subset, which provide a novel target for obesity, obesity-associated diseases including cancer and diabetes treatment.

Consequently, the present invention refers particularly to an ex-vivo method for determining myeloid NK-cells comprising the steps of:
a) providing a sample containing myeloid NK-cells;
b) marking the myeloid NK-cells of the sample by means of at least one marking reagent; and
c) detecting the myeloid NK-cells.

In a preferred embodiment of the invention the ex-vivo method for determining myeloid NK-cells further comprises a step b') after the step b)
b') separating the marked myeloid NK-cells from the sample.

Another preferred embodiment of the invention refers to an ex-vivo method for determining myeloid NK-cells further comprising a step d) after the step c):
d) quantifying the marked myeloid NK-cells.

The myeloid NK-cells, as herein defined, express cell surface marker commonly expressed by NK-cells as well as at least some cell markers typical for cells of the myeloid lineages of blood cells. Some classical cell surface marker of myeloid cells are CD33, CD14 (monocytes), CD68 (macrophages), CD11b, CD11c and CD123 (dentritic cells). It is preferred that the myeloid NK-cells are larger and more granulated compared to mature NK-cells.

Usually, NK-cells express the surface markers CD16 (FcγRIII) and CD56 in humans, NK1.1 or NK1.2 in C57BL/6 mice. The NKp46 cell surface marker is another classical NK-cell marker being expressed in humans, mice and in monkey.

One preferred embodiment of the present invention refers to an ex-vivo method for determining myeloid NK-cells comprising the steps of:
a) providing a sample containing myeloid NK-cells;
b) marking the myeloid NK-cells of the sample by means of at least one marking reagent; and
c) detecting the myeloid NK-cells,
wherein the myeloid NK-cells are characterized by upregulation of at least 50% of the genes selected from the following group consisting of Pla2g7, Fos, Csf1 r, Cd93, Mpeg1, Cybb, Ctss, Spi1, Il6ra, Cd74, Plbd1, Cd14, Clec10a, Il1rn, Sirpa, Pid1, Ptafr, Ly86, Grn, Tgfbi, Ctsh, C1qc, C1qb, Mrc1, Lrp1, Csf2ra, Ncf1, Cxcl9, Cd302, Cd300lb, Nfam1, Trem2, Emilin2, App, Sdc3, Ifi30, Csf2rb, Igsf6, Marcks, Ctsb, Cst3, Hp, Cfp, Lgals3, Cd300ld, Ifngr2, Rasgrp4, Scpep1, Fgd4, Basp1, Ctsz, Slc11a1, Clec12a, Gm2a, Adap2, Msrb1, Trib1, Msr1, Il1b, Klf4, Hck, Acer3, Plekho1, Mafb, Ciita, Axl, Adam15, Mef2c, Cebpb, Cebpa, Dusp1, Ext1, C1qa, Unc93b1, Naaa,

Tmem86a, Lst1, Atf3, Ptpro, Nav1, Pld4, Tlr1, Pou2f2, Lacc1, Themis2, Ccdc109b, Ms4a7, and Rassf4. In other words, the myeloid NK-cells as defined herein are characterized by an increase in the number of the products of at least 50% of the genes selected from the following group consisting of Pla2g7, Fos, Csf1r, Cd93, Mpeg1, Cybb, Ctss, Spi1, Il6ra, Cd74, Plbd1, Cd14, Clec10a, Il1rn, Sirpa, Pid1, Ptafr, Ly86, Grn, Tgfbi, Ctsh, C1qc, C1qb, Mrc1, Lrp1, Csf2ra, Ncf1, Cxcl9, Cd302, Cd300lb, Nfam1, Trem2, Emilin2, App, Sdc3, Ifi30, Csf2rb, Igsf6, Marcks, Ctsb, Cst3, Hp, Cfp, Lgals3, Cd300ld, Ifngr2, Rasgrp4, Scpep1, Fgd4, Basp1, Ctsz, Slc11a1, Clec12a, Gm2a, Adap2, Msrb1, Trib1, Msr1, Il1b, Klf4, Hck, Acer3, Plekho1, Mafb, Ciita, Axl, Adam15, Mef2c, Cebpb, Cebpa, Dusp1, Ext1, C1qa, Unc93b1, Naaa, Tmem86a, Lst1, Atf3, Ptpro, Nav1, Pld4, Tlr1, Pou2f2, Lacc1, Themis2, Ccdc109b, Ms4a7, and Rassf4.

It is even more preferred that at least 60%, 70%, 80%, 85%, 90% or even 95% of these genes are upregulated. In addition, the myeloid NK-cells are preferably defined by downregulation of IL18r1.

Another preferred embodiment of the present invention refers to an ex-vivo method for determining myeloid NK-cells comprising the steps of:
a) providing a sample containing myeloid NK-cells;
b) marking the myeloid NK-cells of the sample by means of at least one marking reagent; and
c) detecting the myeloid NK-cells,
wherein the myeloid NK-cells are characterized by upregulation of at least 50% of the genes selected from the following group consisting of Pla2g7, Fos, Csf1 r, Cd93, Mpeg1, Cybb, Ctss, Spi1, Il6ra, Cd74, Plbd1, Cd14, Clec10a, Il1rn, Sirpa, Pid1, Ptafr, Ly86, Grn, Tgfbi, Ctsh, C1qc, C1qb, Mrc1, Lrp1, Csf2ra, Ncf1, Cxcl9, Cd302, Cd300lb, Nfam1, Trem2, Emilin2, App, Sdc3, Ifi30, Csf2rb, Igsf6, Marcks, Ctsb, and Cst3. It is even more preferred that at least 60%, 70%, 80%, 85%, 90% or even 95% of these genes are upregulated. In addition, the myeloid NK-cells are preferably defined by downregulation of IL18r1.

Most preferably, the present invention refers to an ex-vivo method for determining myeloid NK-cells comprising the steps of:
a) providing a sample containing myeloid NK-cells;
b) marking the myeloid NK-cells of the sample by means of at least one marking reagent; and
c) detecting the myeloid NK-cells,
wherein the myeloid NK-cells are characterized by upregulation of at least Pla2g7, Fos, Csf1r, Cd93, Mpeg1, Cybb, Ctss, Spi1, Il6ra and Cd74. The myeloid NK-cells are preferably further defined by downregulation of IL18r1.

Upregulation means thereby that a change or increase in the amount of the respective gene product by at least factor 1.5, preferably by factor 2, more preferably by factor 5 is detectable, compared to the amount of the respective gene product observed in other (mature) NK-cells (or in further subpopulations of NK-cells). That means the term myeloid NK-cells as used herein refers preferably to NK-cells expressing 1.5, preferably 2, more preferably 5 times more of at least 50% of the genes selected from the following group consisting of Pla2g7, Fos, Csf1r, Cd93, Mpeg1, Cybb, Ctss, Spi1, Il6ra, Cd74, Plbd1, Cd14, Clec10a, Il1rn, Sirpa, Pid1, Ptafr, Ly86, Grn, Tgfbi, Ctsh, C1qc, C1qb, Mrc1, Lrp1, Csf2ra, Ncf1, Cxcl9, Cd302, Cd300lb, Nfam1, Trem2, Emilin2, App, Sdc3, Ifi30, Csf2rb, Igsf6, Marcks, Ctsb, Cst3, Hp, Cfp, Lgals3, Cd300ld, Ifngr2, Rasgrp4, Scpep1, Fgd4, Basp1, Ctsz, Slc11a1, Clec12a, Gm2a, Adap2, Msrb1, Trib1, Msr1, Il1b, Klf4, Hck, Acer3, Plekho1, Mafb, Ciita, Axl, Adam15, Mef2c, Cebpb, Cebpa, Dusp1, Ext1, C1qa, Unc93b1, Naaa, Tmem86a, Lst1, Atf3, Ptpro, Nav1, Pld4, Tlr1, Pou2f2, Lacc1, Themis2, Ccdc109b, Ms4a7, and Rassf4. Downregulation means thereby respectively, that a decrease in the amount of a gene product by at least factor 1.5, preferably by factor 2, more preferably by factor 5 is detectable, compared to the amount of the respective gene product observed in other (classical) NK-cells (or in all further subpopulations of NK-cells). That means the term myeloid NK-cells as used herein refers preferably to NK-cells expressing 1.5, preferably 2, more preferably 5 times or even 10 times less Il18r1, compared to the amount of the respective gene product observed in other (classical) NK-cells (or in all further subpopulations of NK-cells).

One preferred embodiment of the present invention refers to an ex-vivo method for determining myeloid NK-cells comprising the steps of:
a) providing a sample containing myeloid NK-cells;
b) marking the myeloid NK-cells of the sample by means of at least one marking reagent; and
c) detecting the myeloid NK-cells,
wherein the myeloid NK-cells are characterized by a 10-fold upregulation of Pla2g7, a 3-fold upregulation of Fos, a 5-fold upregulation of Csf1r, a 3-fold upregulation of Cd93, a 5-fold upregulation of Mpeg1, a 5-fold upregulation of Cybb, a 3-fold upregulation of Ctss, a 3-fold upregulation of Spi1, a 2-fold upregulation of Il6ra and a 2-fold upregulation of Cd74. The myeloid NK-cells are preferably further defined by a 2-fold downregulation of IL18r1. The upregulation or respectively downregulation is preferably compared to the basal level of the respective gene product observed (measured) in other (classical or mature) NK-cells (or in all further subpopulations of NK-cells).

The sample provided in step a) of the inventive methods is preferably a blood sample, a sample containing enriched blood cells or a tissue sample, in particular, an adipose tissue sample or a tumour sample.

Step b) of the inventive methods, marking the myeloid NK-cells of the sample by means of at least one marking reagent refers preferably to marking of the myeloid NK-cells of the sample by at least two marking reagents recognizing wherein one marking reagent targets a conventional or classical NK-cell marker such as CD56 or NKp46 (in humans) or NK1.1 or NKp46 (in mice) and the second marking reagent targets (at least) one myeloid cell (surface) marker. It is thereby further preferred that the myeloid cell (surface) marker is selected from the group consisting of or comprising Pla2g7, Fos, Csf1r, Cd93, Mpeg1, Cybb, Ctss, Spi1, Il6ra, Cd74, Plbd1, Cd14, Clec10a, Il1rn, Sirpa, Pid1, Ptafr, Ly86, Grn, Tgfbi, Ctsh, C1qc, C1qb, Mrc1, Lrp1, Csf2ra, Ncf1, Cxcl9, Cd302, Cd300lb, Nfam1, Trem2, Emilin2, App, Sdc3, Ifi30, Csf2rb, Igsf6, Marcks, Ctsb, Cst3, Hp, Cfp, Lgals3, Cd300ld, Ifngr2, Rasgrp4, Scpep1, Fgd4, Basp1, Ctsz, Slc11a1, Clec12a, Gm2a, Adap2, Msrb1, Trib1, Msr1, Il1b, Klf4, Hck, Acer3, Plekho1, Mafb, Ciita, Axl, Adam15, Mef2c, Cebpb, Cebpa, Dusp1, Ext1, C1qa, Unc93b1, Naaa, Tmem86a, Lst1, Atf3, Ptpro, Nav1, Pld4, Tlr1, Pou2f2, Lacc1, Themis2, Ccdc109b, Ms4a7, and Rassf4.

It is preferred that the at least one marking reagent is selected from a group consisting of a fluorescent reagent, a dye, an immunostaining reagent, and a radioactive agent, It is advantageous if the marking reagent comprises a portion able to bind NK-cells and is labeled for direct detection (by radioactivity, luminescence, fluorescence, optical or electron density etc.) or indirect detection (e.g., epitope tag such as the FLAG, V5 or *myc* epitopes, an enzyme tag such as horseradish peroxidase or luciferase, a transcription product, etc.). The label may be bound to an antibody recognizing a surface protein of myeloid NK-cells. Thereby high specificity is preferred. Alternatively, several different proteins are labeled by the marking reagent, which represent the specific expression profile of the myeloid NK-cells.

Step b) of the inventive methods read alternatively: labeling the myeloid NK-cell by antibody-mediated labelling of at least one conventional or classical NK-cell marker such as CD56 or NKp46 (in humans) or NK1.1 or NKp46 (in mice) and at least one myeloid cell (surface) marker; preferably simultaneously. It is thereby preferred that the myeloid cell (surface) marker is selected from the group consisting of or comprising Pla2g7, Fos, Csf1r, Cd93, Mpeg1, Cybb, Ctss, Spi1, Il6ra, Cd74, Plbd1, Cd14, Clec10a, Il1rn, Sirpa, Pid1, Ptafr, Ly86, Grn, Tgfbi, Ctsh, C1qc, C1qb, Mrc1, Lrp1, Csf2ra, Ncf1, Cxcl9, Cd302, Cd300lb, Nfam1, Trem2, Emilin2, App, Sdc3, Ifi30, Csf2rb, Igsf6, Marcks, Ctsb, Cst3, Hp, Cfp, Lgals3, Cd300ld, Ifngr2, Rasgrp4, Scpep1, Fgd4, Basp1, Ctsz, Slc11a1, Clec12a, Gm2a, Adap2, Msrb1, Trib1, Msr1, Il1b, Klf4, Hck, Acer3, Plekho1, Mafb, Ciita, Axl, Adam15, Mef2c, Cebpb, Cebpa, Dusp1, Ext1, C1qa, Unc93b1, Naaa, Tmem86a, Lst1, Atf3, Ptpro, Nav1, Pld4, Tlr1, Pou2f2, Lacc1, Themis2, Ccdc109b, Ms4a7, and Rassf4.

Antibodies against surface proteins of the myeloid NK-cells are commercially available or can be prepared by methods known in the art. Examples for suitable labeling groups such as fluorescent groups as well as methods for labeling reactions are known in the art. The marking by a marking reagent can be conveniently checked, using the label for detection.

Another embodiment of the present invention refers to an ex-vivo method for diagnosis of a disease associated with and/or caused by myeloid NK-cells comprising the steps:
a) providing a sample containing myeloid NK-cells;
b) marking the myeloid NK-cells of the sample by means of a marking reagent; and
c) detecting the marked myeloid NK-cells.

One preferred embodiment of the present invention refers to an ex-vivo method for diagnosis of a disease associated with and/or caused by myeloid NK-cells comprising the steps of:
a) providing a sample containing myeloid NK-cells;
b) marking the myeloid NK-cells of the sample by means of at least one marking reagent; and
c) detecting the myeloid NK-cells,
wherein the myeloid NK-cells are characterized by upregulation of at least 50% of the genes selected from the following group consisting of Pla2g7, Fos, Csf1 r, Cd93, Mpeg1, Cybb, Ctss, Spi1, Il6ra, Cd74, Plbd1, Cd14, Clec10a, Il1rn, Sirpa, Pid1, Ptafr, Ly86, Grn, Tgfbi, Ctsh, C1qc, C1qb, Mrc1, Lrp1, Csf2ra, Ncf1, Cxcl9, Cd302, Cd300lb, Nfam1, Trem2, Emilin2, App, Sdc3, Ifi30, Csf2rb, Igsf6, Marcks, Ctsb, Cst3, Hp, Cfp, Lgals3, Cd300ld, Ifngr2, Rasgrp4, Scpep1, Fgd4, Basp1, Ctsz, Slc11a1, Clec12a, Gm2a, Adap2, Msrb1, Trib1, Msr1, Il1b, Klf4, Hck, Acer3, Plekho1, Mafb, Ciita, Axl, Adam15, Mef2c, Cebpb, Cebpa, Dusp1, Ext1, C1qa, Unc93b1, Naaa, Tmem86a, Lst1, Atf3, Ptpro, Nav1, Pld4, Tlr1, Pou2f2, Lacc1, Themis2, Ccdc109b, Ms4a7, and Rassf4.

It is even more preferred that at least 60%, 70%, 80%, 85%, 90% or even 95% of these genes are upregulated. In addition, the myeloid NK-cells are preferably defined by downregulation of IL18r1.

Another preferred embodiment of the present invention refers to an ex-vivo method for diagnosis of a disease associated with and/or caused by myeloid NK-cells comprising the steps of:
a) providing a sample containing myeloid NK-cells;
b) marking the myeloid NK-cells of the sample by means of at least one marking reagent; and
c) detecting the myeloid NK-cells,
wherein the myeloid NK-cells are characterized by upregulation of at least 50% of the genes selected from the following group consisting of Pla2g7, Fos, Csf1 r, Cd93, Mpeg1, Cybb, Ctss, Spi1, Il6ra, Cd74, Plbd1, Cd14, Clec10a, Il1rn, Sirpa, Pid1, Ptafr, Ly86, Grn, Tgfbi, Ctsh, C1qc, C1qb, Mrc1, Lrp1, Csf2ra, Ncf1, Cxcl9, Cd302, Cd300lb, Nfam1, Trem2, Emilin2, App, Sdc3, Ifi30, Csf2rb, Igsf6, Marcks, Ctsb, and Cst3. It is even more preferred that at least 60%, 70%, 80%, 85%, 90% or even 95% of these genes are upregulated. In addition, the myeloid NK-cells are preferably defined by downregulation of IL18r1.

Most preferably, the present invention refers to an ex-vivo method for diagnosis of a disease associated with and/or caused by myeloid NK-cells comprising the steps of:
a) providing a sample containing myeloid NK-cells;
b) marking the myeloid NK-cells of the sample by means of at least one marking reagent; and
c) detecting the myeloid NK-cells,
wherein the myeloid NK-cells are characterized by upregulation of at least Pla2g7, Fos, Csf1r, Cd93, Mpeg1, Cybb, Ctss, Spi1, Il6ra and Cd74. The myeloid NK-cells are preferably further defined by downregulation of IL18r1.

Upregulation and downregulation is thereby defined as previously mentioned. One preferred embodiment of the present invention refers to an ex-vivo method for diagnosis of a disease associated with and/or caused by myeloid NK-cells comprising the steps of:
a) providing a sample containing myeloid NK-cells;
b) marking the myeloid NK-cells of the sample by means of at least one marking reagent; and
c) detecting the myeloid NK-cells,
wherein the myeloid NK-cells are characterized by a 10-fold upregulation of Pla2g7, a 3-fold upregulation of Fos, a 5-fold upregulation of Csf1r, a 3-fold upregulation of Cd93, a 5-fold upregulation of Mpeg1, a 5-fold upregulation of Cybb, a 3-fold upregulation of Ctss, a 3-fold upregulation of Spi1, a 2-fold upregulation of Il6ra and a 2-fold upregulation of Cd74. The myeloid NK-cells are preferably further defined by a 2-fold downregulation of IL18r1. The upregulation or respectively downregulation is preferably compared to the basal level of the respective gene product observed (measured) in other (classical or mature) NK-cells (or in all further subpopulations of NK-cells).

One preferred embodiment of the present invention relates to ex-vivo method for diagnosis of a disease associated with and/or caused by myeloid NK-cells, further comprising steps d) - e) after the step c):
d) quantifying the marked myeloid NK-cells; and
e) comparing the quantified value of the marked myeloid differentiated NK-cells with a standard value.
Quantification of the marked myeloid NK-cells may be done by measuring the signal produced by the marking reagent, such as fluorescence. Alternatively, cell sorting can be used. However, several methods are known in the art how to quantify a subpopulation of blood cells having a specific expression profile, especially an expression of cell surface marker. It is further common knowledge that determining a change in a signal, such as fluorescence decrease or increase, here in general the signal produced by the marking reagent is only possible if a sample of known amount of myeloid NK-cells is measured, too. Therefore, the inventive method may comprise step e) comparing the quantified value of the marked myeloid NK-cells with a standard value. Alternatively, a reference sample, such as a population known not to contain myeloid NK-cells, is measured too, and the resulting value is used in step e).

It is preferred that the disease associated with and/or caused by myeloid NK-cells is selected from the group comprising or consisting of obesity, insulin resistance, diabetes (preferably diabetes type 2), autoimmune diseases, (obesity-associated) cancer, chronic infections and inflammation. One embodiment of the invention relates to a method for the diagnosis of of a disease associated with and/or caused by myeloid NK-cells and/or caused by myeloid NK-cells, comprising: providing a sample containing myeloid NK-cells, preferably a blood or adipose tissue, from a subject suspected to suffer from obesity, insulin resistance, diabetes (preferably diabetes type 2), autoimmune diseases, (obesity-associated) cancer, chronic infections and inflammation.

One preferred embodiment of the present invention relates to the ex-vivo method for diagnosis of a disease associated with and/or caused by myeloid NK-cells and/or caused by myeloid NK-cells, wherein that disease is obesity or induced diabetes, particularly, diabetes induced by high fat diet.

Another preferred embodiment of the present invention relates to the ex-vivo method for diagnosis of a disease associated with and/or caused by myeloid NK-cells and/or caused by myeloid NK-cells, wherein the disease is inflammation, in particular, obesity-induced inflammation or metaflammation.

A further preferred embodiment of the present invention relates to the ex-vivo method for diagnosis of a disease associated with and/or caused by myeloid NK-cells and/or caused by myeloid NK-cells, further comprising a step f):
f) detecting and quantifying STAT3 and p-STAT3 (phosphorylated STAT3) of the sample.

Another aspect of the present invention refers to a method for determining whether a candidate agent reduces or inhibits a myeloid NK-cell population comprising:
a) providing a sample containing the myeloid NK-cell population;
b) contacting the sample containing the myeloid NK-cell population with the candidate agent;
c) marking the myeloid NK-cell population of the sample by means of a marking reagent;
d) detecting the myeloid NK-cell population; and
e) determining if the candidate agent reduces or inhibits the myeloid NK-cell population.

That screening method of the present invention apparently consists of five steps. The sample provided in step a) of the inventive methods is preferably a blood sample, a sample containing enriched blood cells. Concerning that screening method, the sample may also be a cell culture of NK-cells or myeloid NK-cells.

Contacting of the sample containing the myeloid NK-cell population with the candidate agent can happen e.g. in the form of a compound library, in physiological or non-physiological solution, or solid phase systems, however a liquid environment, particularly, whole blood or cell culture medium is preferred. The conditions and the time needed to be sufficient to allow the candidate agent to affect the myeloid NK-cell population varies dependent on the set-up but is preferably between 15 minutes and 72 hours. Nevertheless, the method is usally carried out in solution, around 37°C and at a suitable pH value about pH 7.4. The time needed comprises up to several cell cycles of the NK-cells. All parameters are easily selected by a skilled person.

The tested candidate agent reduces a myeloid NK-cell population if compared to a sample of untreated myeloid NK-cells the number of myeloid NK-cells is reduced after incubation with the candidate agent. The tested candidate agent inhibits a myeloid NK-cell population if the treated cells grow slower, secrete less cytokines or if at least one cell pathway of these cells is inhibited.

It is preferred that the inventive method for determining whether a candidate agent reduces a myeloid NK-cell population refers to myeloid NK-cells that are characterized by upregulation of at least 50% of the genes selected from the following group consisting of Pla2g7, Fos, Csf1r, Cd93, Mpeg1, Cybb, Ctss, Spi1, Il6ra, Cd74, Plbd1, Cd14, Clec10a, Il1rn, Sirpa, Pid1, Ptafr, Ly86, Grn, Tgfbi, Ctsh, C1qc, C1qb, Mrc1, Lrp1, Csf2ra, Ncf1, Cxcl9, Cd302, Cd300lb, Nfam1, Trem2, Emilin2, App, Sdc3, Ifi30, Csf2rb, Igsf6, Marcks, Ctsb, Cst3, Hp, Cfp, Lgals3, Cd300ld, Ifngr2, Rasgrp4, Scpep1, Fgd4, Basp1, Ctsz, Slc11a1, Clec12a, Gm2a, Adap2, Msrb1, Trib1, Msr1, Il1b, Klf4, Hck, Acer3, Plekho1, Mafb, Ciita, Axl, Adam15, Mef2c, Cebpb, Cebpa, Dusp1, Ext1, C1qa, Unc93b1, Naaa, Tmem86a, Lst1, Atf3, Ptpro, Nav1, Pld4, Tlr1, Pou2f2, Lacc1, Themis2, Ccdc109b, Ms4a7, and Rassf4. It is even more preferred that at least 60%, 70%, 80%, 85%, 90% or even 95% of these genes are upregulated. In addition, the myeloid NK-cells are preferably defined by downregulation of IL18r1. Upregulation and downregulation is thereby defined as previously mentioned. Most preferably, the myeloid NK-cells are characterized by a 10-fold upregulation of Pla2g7, a 3-fold upregulation of Fos, a 5-fold upregulation of Csf1r, a 3-fold upregulation of Cd93, a 5-fold upregulation of Mpeg1, a 5-fold upregulation of Cybb, a 3-fold upregulation of Ctss, a 3-fold upregulation of Spi1, a 2-fold upregulation of Il6ra and a 2-fold upregulation of Cd74. The myeloid NK-cells are preferably further defined by a 2 fold downregulation of IL18r1.

A preferred embodiment of the present invention refers to a method for determining whether a candidate agent reduces a myeloid NK-cell population, wherein the candidate agent is a small organic non-peptidic molecule, a peptidic compound, a nucleic acid, or a metal complex.

A candidate agent reduces a myeloid NK-cell population may be selected from:
(i) nucleic acids, in particular small interfering RNA (siRNA), micro RNA (miRNA) or a precursor thereof, oligonucleotide aptamers, anti-sense oligonucleotides, or ribozymes;
(ii) peptidic compounds, in particular proteins, like signaling molecules, antibodies, antibody fragments or peptidic aptamers;
(iii) small organic non-peptidic molecules, i.e. molecules having a low molecular weight; and
(iv) combinations thereof.

Such an agent or compound may have the ability to influence, in particular, to inhibit IL6-signaling or the downstream signaling pathway which converts on Stat3. In one embodiment, the candidate agent is able to bind a target polypeptide, i.e. an enzyme acting on the protein level, such as a peptide acting as a ligand on IL6 receptor and thereby inhibiting IL6 pathway.

A candidate agent may also be an antibody binding and influencing at least one specific target polypeptide. In the context of the present invention, the term "antibody" covers monoclonal antibodies, polyclonal antibodies, multispecific antibodies (e.g. bispecific antibodies) formed from at least two antibodies, antibody fragments and derivatives thereof if they exhibit the desired activity. The antibody may be an IgM, IgG, e.g. IgG1, IgG2, IgG3 or IgG4. Antibody fragments comprise a portion of an antibody, generally the antigen binding or variable region of the intact antibody. Examples of antibody fragments include Fab, Fab', F (ab') 2 and Fv fragments, diabodies, single chain antibody molecules and multispecific antibody fragments. A bispecific monoclonal antibody (BsMAb, BsAb) is an artificial protein made from two different monoclonal antibodies that can bind to two different types of antigen simultaneously.

Particularly, the antibody may be a recombinant antibody or antibody fragment, more particularly selected from chimeric antibodies or fragments thereof and diabodies. For therapeutic purposes, particularly for the treatment of humans, the administration of chimeric antibodies, humanized antibodies or human antibodies is especially preferred. A monoclonal antibody may be obtained by the hybridoma method as described by Köhler et al. (Nature 256 (1975), 495) or by recombinant DNA methods (cf. e.g. US patent 4,816,567). Monoclonal antibodies may also be isolated from phage antibody libraries using techniques which are known to the person skilled in the art.

Further, candidate agent may be a small organic non-peptidic molecule. Such a molecule has a low molecular weight between 100 and 1000 g·mol⁻¹ and preferred between 300 and 500 g·mol⁻¹. Said molecule may act as an inhibitor of an enzymatic activity, e.g. a kinase, such as JAK1/2. The term small molecule refers to low molecular weight organic compound which is by definition not a polymer. In the field of pharmacology, it is usually restricted to a molecule that also binds with high affinity to a biopolymer such as proteins, nucleic acids, or polysaccharides. Small molecules are broadly used as enzyme inhibitors.

In a further embodiment, the candidate agent according to the invention is directed against a target nucleic acid, i.e. factor acting on the nucleic acid level. Preferably, the factor is a nucleic acid compound, e.g. RNAi inducing molecules like siRNA, miRNA, anti-sense oligonucleotide, ribozyme, a precursor or a combination thereof.

A RNAi-inducing molecule may refer to a nucleic acid molecule, wherein at least one polynucleotide strand of said nucleic acid molecule has a sequence which is sufficiently complementary to a target RNA, preferably to a target mRNA, in order to affect its processing, i.e. its decomposition. To have an RNAi-inducing effect, it is necessary that the complementarity between the RNAi-inducing molecule and a region of the target RNA is sufficient, to affect a hybridization and a subsequent processing. For example, the complementarity is at least 80 %, preferably at least 90 % and most preferably at least 99 %, whereby the 5'- and/or 3'-ends as well as the overhangs of an RNAi-effector molecule may also contain nucleotides which are not complementary to the target RNA.

SiRNA (small interfering RNA or short interfering RNA or silencing RNA) used according to the invention is a double-strand of RNA and/or nucleotide analogues with 3' overhangs on at least one end, preferably either ends. Each RNA strand of the double-strand has a 5' phosphate group and a 3' hydroxyl group. Preferably, each RNA strand of the double strand is 19 to 30 nucleotides long, more preferably 20 to 28 nucleotides and most preferably 21 to 23 nucleotides. In a particularly preferred embodiment, the siRNA double-strand consists of two 21 nucleotides long RNA strands each having a 3' overhang. SiRNA molecules further refer to single-stranded RNA-molecules having a length of 19-30 nucleotides, preferably 20-28 nucleotides and particularly having a length of 21-23 nucleotides, whereby the single-stranded RNA molecule is for at least 80%, preferably for at least 90% and more preferably for more than 99% complementary to a sequence of a target RNA, in particular of a target mRNA, and a binding of siRNA to the target RNA effects a sequence specific decrease. Preferably, siRNA molecules have overhangs of 1-3 nucleotides on the 3' end. Methods for obtaining siRNA molecules are known to the person skilled in the art.

MiRNA is a single- or double-stranded RNA molecule of 19-30, preferably 20-28, and more preferably 21-23 nucleotides in length, which can regulate gene expression. MiRNA is generally synthesized at first as a precursor, which is then processed to the major form having a sequence which is at least partially complementary to messenger RNA of a target molecule according to the invention.

An antisense oligonucleotide may be a single, double, or triple-stranded DNA, RNA, PNA (peptide nucleic acid) or a combination thereof (e.g. hybrids of DNA and RNA strands) having a length of between about 10-100, preferably 20-50, and more preferably 20-30 nucleotides in length, which can interfere with mRNA targets by hybrid formation and therefore inhibit translation of said mRNA.

Ribozymes are catalytic RNAs which possess a well defined structure that enables them to catalyze a chemical reaction. Apart from naturally occurring ribozymes they can be made artificially and be tailored to interact with nucleic acids and proteins. Ribozymes are also preferred factors for inhibition of the preferred kinases in the present invention.

Precursor molecules, e.g. precursor molecules of siRNA and/or miRNA may be a substrate for the siRNA/miRNA-biogenesis-apparatus of the target cell. This comprises, for example, RNA precursor molecules such as double-stranded RNA (dsRNA) or short hairpin RNA-molecules (shRNA), which are processed by endoribonucleases such as Drosha and/or Pasha to siRNA-molecules or miRNA-molecules, respectively. Dicer is another endoribonuclease that cleaves double-stranded RNA and pre-microRNA (miRNA) into siRNA about 20-25 nucleotides long, usually with a two-base overhang on the 3' end. Dicer catalyzes the first step in the RNA interference pathway and initiates formation of the RNA-induced silencing complex (RISC). The RISC complex with a bound siRNA recognizes complementary messenger RNA (mRNA) molecules and degrades them, resulting in substantially decreased levels of protein translation and effectively turning off the gene.

DsRNA-molecules or short hairpin RNA-molecules (shRNA) having a length of more than 27 nucleotides, preferably more than 30 up to 100 nucleotides or longer, and mostly preferred dsRNA-molecules having a length of 30-50 nucleotides, can be used.

One preferred embodiment of the present invention relates to ex-vivo method for diagnosis of a disease associated with and/or caused by myeloid NK-cells, wherein step e) reads: e) determining if the candidate agent inhibits activity of myeloid NK-cells.

Another preferred embodiment of the present invention relates to ex-vivo method for diagnosis of a disease associated with and/or caused by myeloid NK-cells, wherein step e) includes determining if the candidate agent modulates (preferably inhibits) the activity of at least one protein of a group comprising or consisting of IL6Ra, Csf1r, gp130, JAK1/2, Spi1 and STAT3. This modulation may be an increase or a decrease in signaling activity, binding characteristics, functional, or any other biological property of the polypeptide.

The term candidate agent as it appears herein refers inter alia to a molecule that is able to change or regulate or modulate the activity of myeloid NK-cell. Alternatively, the candidate agent may regulate (preferably decrease) the number of myeloid NK-cells.

In another preferred embodiment, the action of IL6Ra, Csf1r, gp130, JAK1/2 STAT3, SPi1 and other transcription factors of the group of upregulated myeloid genes is impeded by interference to its respective nucleic acid, which can be both DNA and RNA, by inactivation, degradation, downregulation, or intercalation. Inactivation of a nucleic acid can happen for instance by methylation of nucleotides, insertion, deletion, nucleotide exchange, cross linkage, or strand break/damage. Downregulation of DNA or RNA is referred to as diminished expression of these nucleic acids and can happen by binding of repressors, which are usually polypeptides, but can also happen by chemical or structural changes or modifications of the nucleic acids. Intercalation is the reversible inclusion of a molecule between two other molecules. In nucleic acids, intercalation occurs when ligands of an appropriate size and chemical nature fit themselves in between base pairs.

A further aspect of the present invention is the depletion or inhibition of formation of myeloid NK-cells for use in treating obesity, insulin resistance, diabetes, hyperlipidemia, autoimmune diseases, (obesity-associated) cancer, chronic infections or inflammation. Thereby diabetes refers to diabetes type 1, diabetes type 2, MODY, and gestational diabetes but preferred is diabetes type 2, obesity induced diabetes, and particularly, diabetes induced by high fat diet.

One preferred embodiment of the present invention refers to the depletion or inhibition of myeloid NK-cells for use in the treatment of obesity, insulin resistance, diabetes, hyperlipidemia, autoimmune diseases, (obesity-associated) cancer, chronic infections or inflammation including depleting or inhibiting the myeloid NK-cells using antibodies complementary to or binding to at least one of the common NK-cell marker (CD 56 and/or NKp46) and to at least one cell marker selected from the group comprising or consisting of Pla2g7, Fos, Csf1r, Cd93, Mpeg1, Cybb, Ctss, Spi1, Il6ra, Cd74, Plbd1, Cd14, Clec10a, Il1rn, Sirpa, Pid1, Ptafr, Ly86, Grn, Tgfbi, Ctsh, C1qc, C1qb, Mrc1, Lrp1, Csf2ra, Ncf1, Cxcl9, Cd302, Cd300lb, Nfam1, Trem2, Emilin2, App, Sdc3, Ifi30, Csf2rb, Igsf6, Marcks, Ctsb, Cst3, Hp, Cfp, Lgals3, Cd300ld, Ifngr2, Rasgrp4, Scpep1, Fgd4, Basp1, Ctsz, Slc11a1, Clec12a, Gm2a, Adap2, Msrb1, Trib1, Msr1, Il1b, Klf4, Hck, Acer3, Plekho1, Mafb, Ciita, Axl, Adam15, Mef2c, Cebpb, Cebpa, Dusp1, Ext1, C1qa, Unc93b1, Naaa, Tmem86a, Lst1, Atf3, Ptpro, Nav1, Pld4, Tlr1, Pou2f2, Lacc1, Themis2, Ccdc109b, Ms4a7, and Rassf4. Thereby, it is preferred that at least two antibodies are used, one complementary to or binding to at least one of the common NK-cell marker (CD 56 and/or NKp46) and the other antibody is complementary or binds to one cell marker selected from the group comprising or consisting of Pla2g7, Fos, Csf1r, Cd93, Mpeg1, Cybb, Ctss, Spi1, Il6ra, Cd74, Plbd1, Cd14, Clec10a, Il1rn, Sirpa, Pid1, Ptafr, Ly86, Grn, Tgfbi, Ctsh, C1qc, C1qb, Mrc1, Lrp1, Csf2ra, Ncf1, Cxcl9, Cd302, Cd300lb, Nfam1, Trem2, Emilin2, App, Sdc3, Ifi30, Csf2rb, Igsf6, Marcks, Ctsb, Cst3, Hp, Cfp, Lgals3, Cd300ld, Ifngr2, Rasgrp4, Scpep1, Fgd4, Basp1, Ctsz, Slc11a1, Clec12a, Gm2a, Adap2, Msrb1, Trib1, Msr1, Il1b, Klf4, Hck, Acer3, Plekho1, Mafb, Ciita, Axl, Adam15, Mef2c, Cebpb, Cebpa, Dusp1, Ext1, C1qa, Unc93b1, Naaa, Tmem86a, Lst1, Atf3, Ptpro, Nav1, Pld4, Tlr1, Pou2f2, Lacc1, Themis2, Ccdc109b, Ms4a7, and Rassf4. Nevertheless, it is also possible to use bispecific antibodies.

Treatment, as used herein, includes also prevention. It may be advantages to deplete or inhibit formation of myeloid NK-cells in patients suspected to develop obesity, insulin resistance, diabetes, hyperlipidemia, autoimmune diseases, (obesity-associated) cancer, chronic infections or inflammation. This can be because of family background or genetic alterations known.

It is preferred that depletion or inhibition of formation of myeloid NK-cells is mediated by inhibitors of a cell signaling pathway activated in myeloid NK-cells, such as the IL6 signaling pathway such as JAK1/2 and/or Stat3 inhibitors. It is preferred that the inhibited signaling pathway is activated in the myeloid NK-cells but not or less activated in other (mature) NK-cells. Therefore, one preferred embodiment of the present invention refers to inhibitors of the IL6 signaling pathway such as JAK1/2 and/or Stat3 inhibitors for use in treating obesity, insulin resistance, diabetes, or autoimmune diseases, (obesity-associated) cancer, chronic infections or inflammation. Thereby it is preferred that the Inhibitor of JAK1/2 and/or Stat3 is selected from the group comprising or consisting of ruxolitinib, *AZD9150, and PLX3397.*

Consequently, one embodiment of the present invention refers to the depletion or inhibition of myeloid NK-cells for use in medicine and preferably for use in the treatment of obesity, insulin resistance, diabetes, hyperlipidemia, autoimmune diseases, (obesity-associated) cancer, chronic infections or inflammation by inhibition or downregulation of one factor selected from the group comprising or consisting of TREM1, OLR1, CEBPA, SPI1, LRP1, IL6R, CSF1R, C5AR1, TREM2, C3AR1, CLEC7A, CCR1, DDR1, TLR9, TLR2, MERTK, IGF1, CD36, TLR7, CCND1, ID1, MET, KLF4, ENG, ID3, CD40, ADORA2B, BTK, BTNL2, CEBPB, ADGRF5, FOS, IRF5, CAV1, CDKN2A, E2F1, MYBL2, SEMA7A, FOXM1, PTPRJ, DUSP1, SPIB, and CHEK1.

For use in medicine or as a medicament, the agent suitable for depletion or inhibition of formation of myeloid NK-cells according to the invention may be formulated as a pharmaceutical composition. Thus, another aspect of the present invention is directed to pharmaceutical compositions comprising or consisting of an effective amount of at least one agent suitable for depletion or inhibition of formation of myeloid NK-cells, and at least one pharmaceutically acceptable carrier, excipient, binders, disintegrates, glidents, diluents, lubricants, coloring agents, sweetening agents, flavouring agents, preservatives, solvent or the like. Alternatively, an agent for inactivation of myeloid NK-cells is suitable in medicine or as a medicament, in particular for the treatment of diseases of the group comprising or consisting of obesity, insulin resistance, diabetes, hyperlipidemia, autoimmune diseases, (obesity-associated) cancer, chronic infections or inflammation. Also, such an agent may be formulated as a pharmaceutical composition. Thus, another aspect of the present invention is directed to pharmaceutical compositions comprising or consisting of an effective amount of at least one agent suitable for inactivation of myeloid NK-cells, and at least one pharmaceutically acceptable carrier, excipient, binders, disintegrates, glidents, diluents, lubricants, coloring agents, sweetening agents, or flavouring agents may be formulated as a pharmaceutical composition. The pharmaceutical compositions of the present invention can be prepared in a conventional solid or liquid carrier or diluents and a conventional pharmaceutically-made adjuvant at suitable dosage level in a known way.

According to the invention, the pharmaceutical composition can be used for the treatment of diseases of the group comprising or consisting of obesity, insulin resistance, diabetes, hyperlipidemia, autoimmune diseases, (obesity-associated) cancer, chronic infections or inflammation.

The pharmaceutical compositions of the present invention can be prepared in a conventional solid or liquid carrier or diluent at suitable dosage level in a known way. The preferred preparations are adapted for oral application. These administration forms include, for example, pills, tablets, film tablets, coated tablets, capsules, powders and deposits. Another preferred preparation is adapted for injection

Furthermore, the present invention also includes pharmaceutical preparations for parenteral application, including dermal, intradermal, intragastral, intracutan, intravasal, intravenous, intramuscular, intraperitoneal, intranasal, intravaginal, intrabuccal, percutan, rectal, subcutaneous, sublingual, topical, or transdermal application, which preparations in addition to typical vehicles and/or diluents contain at least one compound according to the present invention and/or a pharmaceutical acceptable salt thereof as active ingredient.

The pharmaceutical compositions according to the present will typically be administered together with suitable carrier materials selected with respect to the intended form of administration, i.e. for oral administration in the form of tablets, capsules (either solid filled, semi-solid filled or liquid filled), powders for constitution, extrudates, deposits, gels, elixirs, dispersible granules, syrups, suspensions, and the like, and consistent with conventional pharmaceutical practices. For example, for oral administration in the form of tablets or capsules, the active drug component may be combined with any oral non-toxic pharmaceutically acceptable carrier, preferably with an inert carrier like lactose, starch, sucrose, cellulose, magnesium stearate, dicalcium phosphate, calcium sulfate, talc, mannitol, ethyl alcohol (liquid filled capsules) and the like. Moreover, suitable binders, lubricants, disintegrating agents and coloring agents may also be incorporated into the tablet or capsule.

The mentioned pharmaceutical formulations are characterized in that they comprise a compound able to deplete myeloid NK-cells or to inhibit the formation of myeloid NK-cells. This compound is present in said pharmaceutical formulation in the range of 1 to 1000 µg/g. In a preferred embodiment of the invention the compound is present in said formulation in the range of 10 to 1000 ng/g.

### Description of the Figures

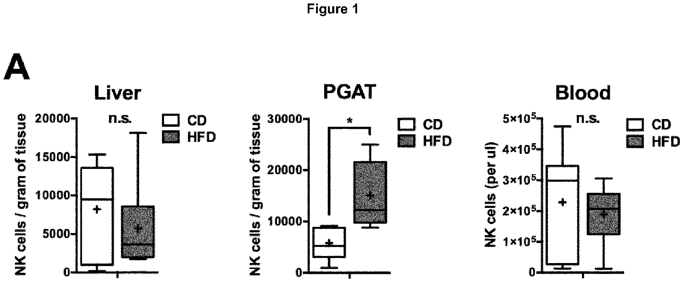

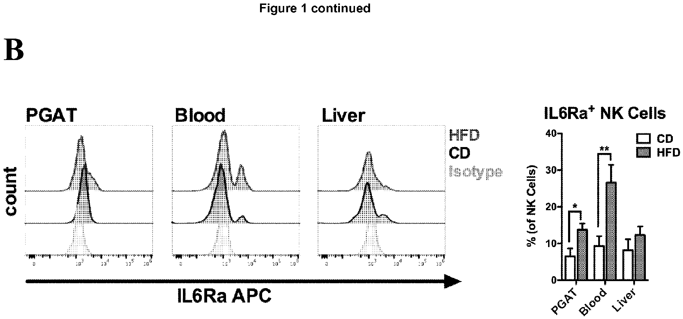

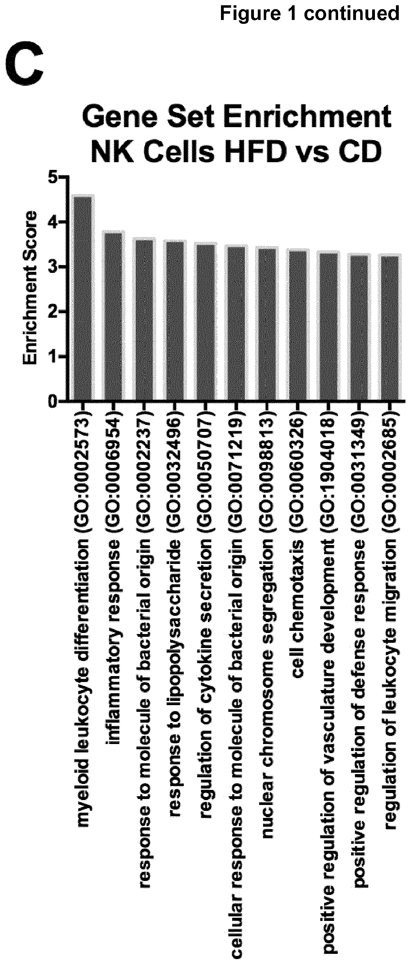

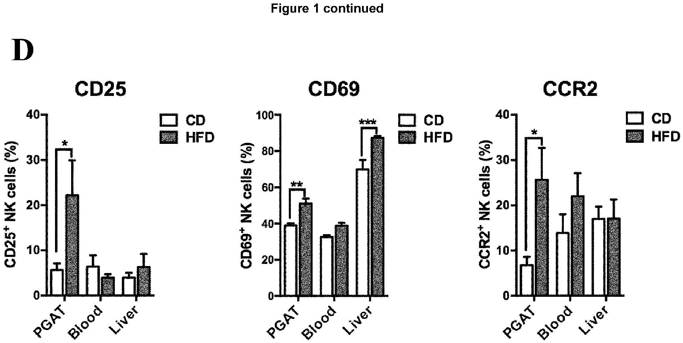

**Fig. 1****: NK cells in obese C57/BI6 wildtype mice upregulate IL6Ra expression** Non-parenchymal cells were isolated from PGAT, blood and liver, and NK cells were defined as CD45⁺ CD3⁻ NK1.1⁺ Ncr1⁺ viable, single cells via flow cytometry.
   **A)** Absolute numbers of NK cells per organ mass or blood volume are shown from PGAT, blood and liver of BI6 wildtype mice after 16 weeks of HFD- or CD-feeding (n=18vs18).
   **B)** Flow cytometric analysis of IL6Ra expression on NK cells derived from organs of obese versus lean mice after 16 weeks of HFD-feeding. Histograms show representative results. Quantification of IL6Ra⁺ NK cells (defined as viable, single CD45⁺CD3⁻NK1.1⁺ cells, n=6vs8).
   **C)** Gene set enrichment analysis of transcriptomes in PGAT derived NK cells of obese versus lean mice. The most significantly enriched gene ontologies are shown.
   **D)** Quantification of CD25⁺, CD69⁺ and CCR2⁺ NK cells in liver, PGAT and blood (n=6vs8). (Statistics: unpaired 2-sided t-test corrected for multiple testing; n.s., not significant; *p ≤0.05, **p ≤0.01, ***p ≤0.001)

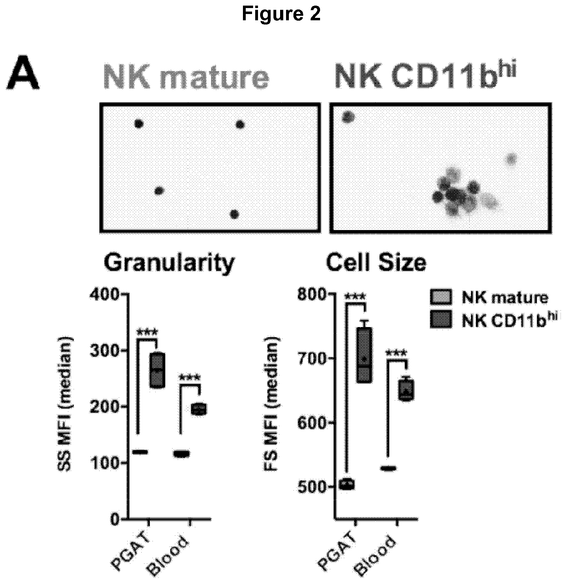

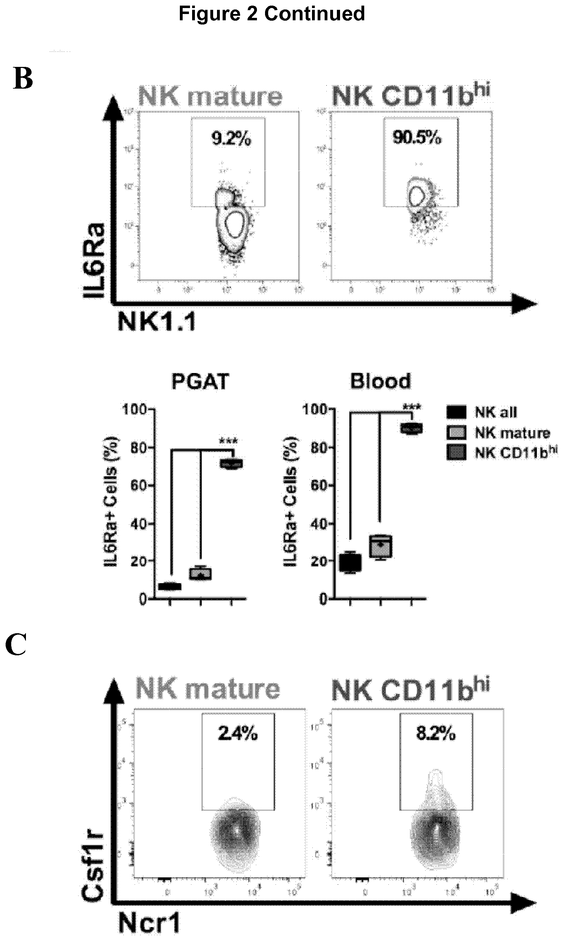

**Fig. 2****: CD11b^{hi} NK-cells are morphological distinct from mature NK-cells and express myeloid as well as activated NK-cell gene sets** CD11b^{hi} and mature CD11b⁺ NK-cells were FACS-purified from PGAT and blood to perform quantitative gene expression analysis via mRNA deep-sequencing.
   **A)** Cytomorphology of Pappenheim-stained CD11b^{hi} and mature NK-cells from blood directly sorted onto glass slides (630-fold magnification) and, lower part, flow-cytometric quantification of the cell size and granularity by median fluorescence intensity (MFI) of forward (FS) and sideward (SS) scatters (n=4vs4) (statistics: unpaired 2-sided t-test; ***p ≤0.001).
   **B)** Flow cytometric analysis of IL6Ra-expression in mature and CD11b^{hi} NK-cells derived from PGAT or blood. Dot blots either gated on mature (left) or CD11b^{hi} (right) NK-cells show representative results. Cumulative quantification shown as percent of IL6Ra⁺ NK-cells of the respective parental population (n=4) (statistics: unpaired 2-sided t-test with Bonferroni multiple comparison correction; ***p ≤0.001).
   **C)** Exemplary flow cytometry zebra blots of Csf1r expression gated either on mature, i.e. CD11b⁺, (left) or CD11b^{hi} (right) NK-cells.

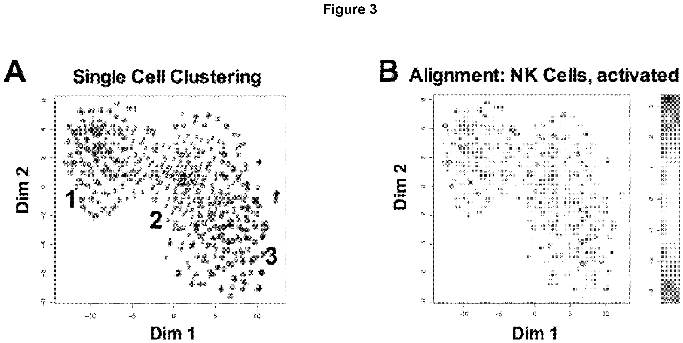

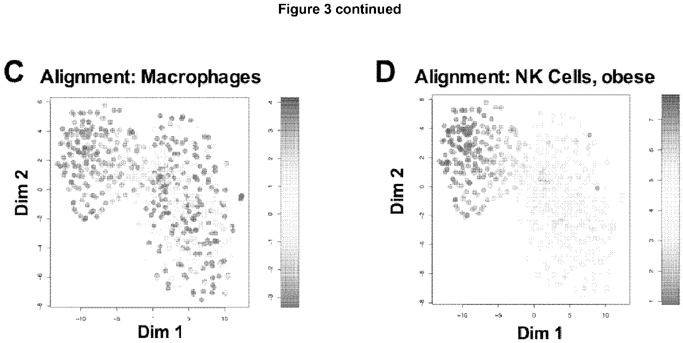

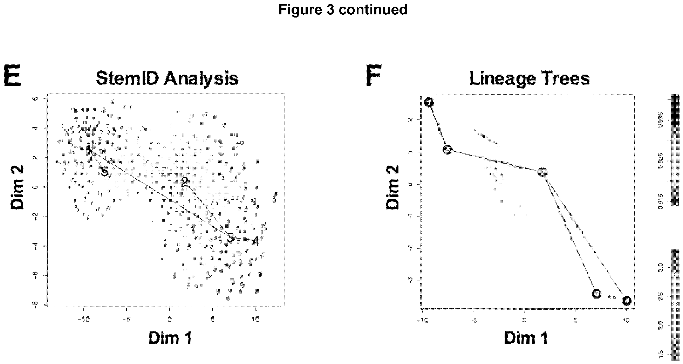

**Figure 3****: Single cell sequencing and transcriptome analysis identifies formation of a distinct, obesity-associated NK-cell population in adipose tissue of obese mice.**
   Single, viable CD45⁺CD3⁻NK1.1⁺CD11b⁺ NK-cells were isolated from PGAT of lean or obese wildtype C57/BI6 mice and a total of 768 single cells were individually sorted.
   **A)** Unsupervised cluster analysis (tSNE map) of individually cells based on their gene expression patterns results in three clusters (blue=cluster 1; green= cluster 2; purple= cluster 3).
   **B)** Alignment of a published gene signature (Bezman et al., Nat. Immunol 2012) of late activated NK-cells (expression in log scale).
   **C)** Alignment of a publically available signature (www.immgen.org) of genes expressed in macrophages and
   **D)** alignment of genes up-regulated in bulk sequenced NK-cells from adipose tissue of obese mice (expression in log scale).
   **E)** StemID analysis based on the same tSNE map as shown in A), which only shows 3 clusters, however the outlier cells in the clusters (the ones, which fit the least into the cluster) get their own cluster (cluster 5 and 4). StemID runs on the dataset including these outlier clusters. The black lines reflect a minimum spanning tree for the cluster medoids.
   **F)** Lineage tree analysis shows the projections of all cells in t-SNE space. The color of the links indicates the -log10p-value of the link and the color of the vertices indicates the delta-entropy. The width of the connections in the right panel indicates the link score. Line width corresponds to link score multiplied by 5.

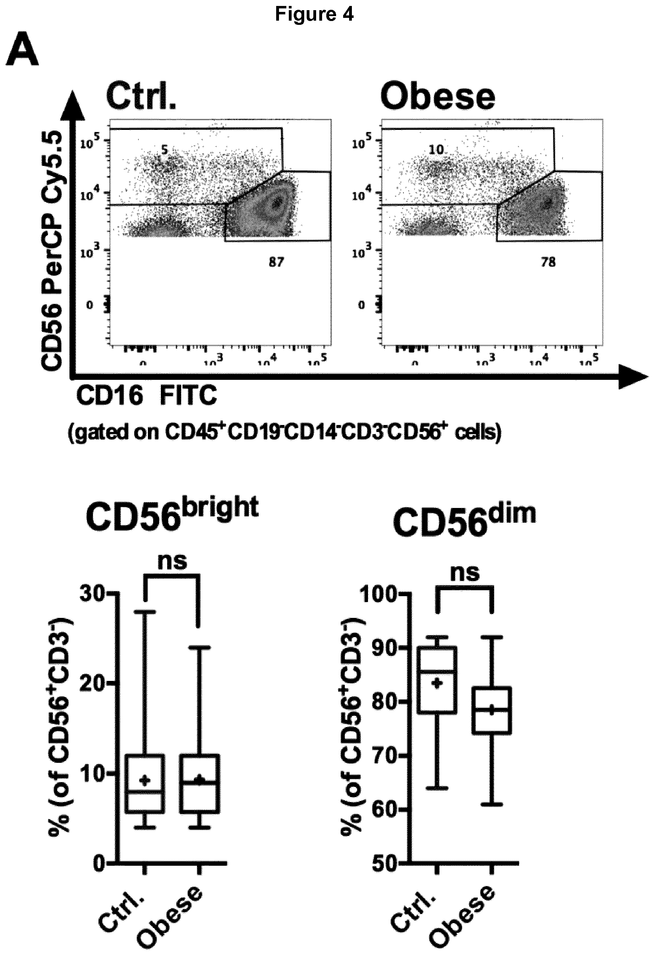

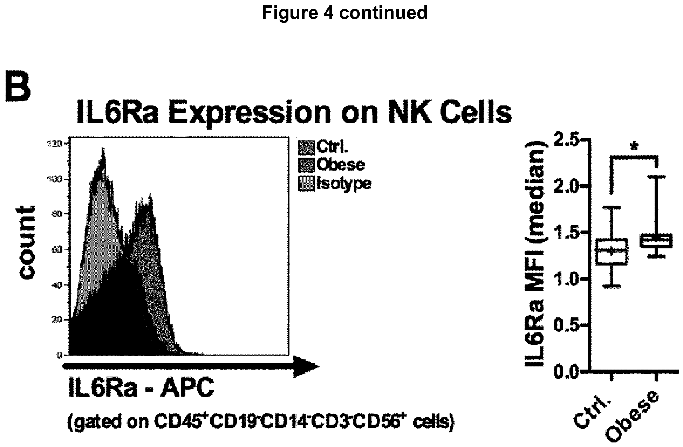

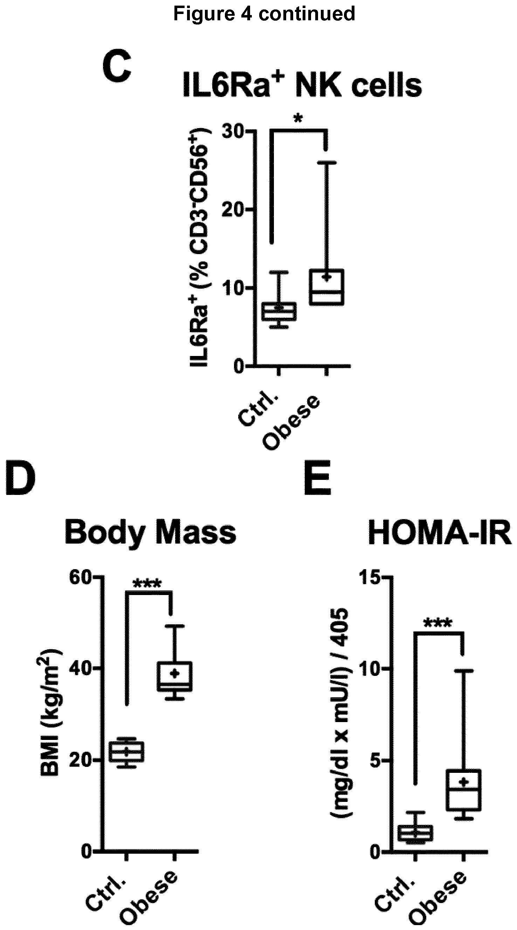

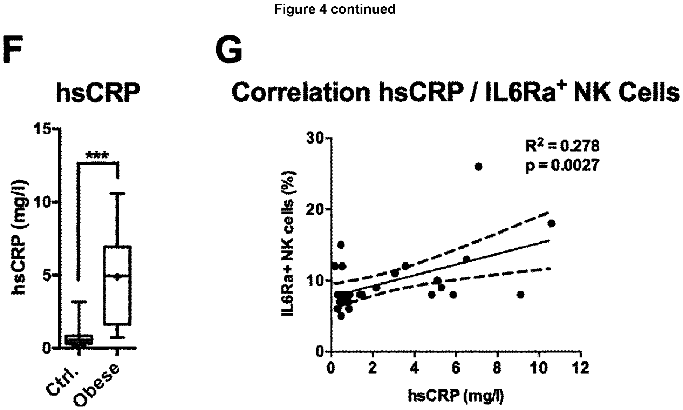

**Fig. 4****: Circulating IL6Ra⁺ NK-cells are detectable in obese humans and correlate with markers of metaflammation.**
   NK-cells defined as viable, single CD45⁺ lin^{- (CD3-CD14-CD19-)} CD56⁺ cells were analyzed in peripheral blood mononuclear cells (PBMC) of lean (ctrl.) and obese humans by flow cytometry.
   **A)** Representative flow cytometry dot plots of classical CD56^{bright} and CD56^{dim} NK-cell subpopulations in a ctrl. and obese individual. Quantification of CD56^{bright} and CD56^{dim} NK-cell subpopulations within the entire study cohort (ctrl. n=16; obese n=14).
   **B)** Analysis of IL6Ra expression in CD3⁻CD56⁺ NK-cells exemplarily shown in a control and an obese individual. Quantification of IL6Ra expression as median fluorescence intensity (MFI) in CD3⁻CD56⁺ NK-cells.
   **C)** Relative numbers of IL6Ra⁺ NK-cells (in % of CD3⁻CD56⁺ cells).
   **D-F)** Clinical data and blood samples from obese (n=14) and lean (n=16) subjects were prospectively collected and comprised
      **D)** the body-mass-index,
      **E)** the homeostatic model assessment of insulin resistance (HOMA-IR) as a measure of insulin sensitivity, and
      **F)** plasma concentrations of high-sensitivity C-reactive protein (hsCRP) as a measure of systemic low-grade inflammation.
   **G)** Linear regression analysis of hsCRP levels and the number of circulating IL6Ra⁺ NK-cells in each lean and obese individual.

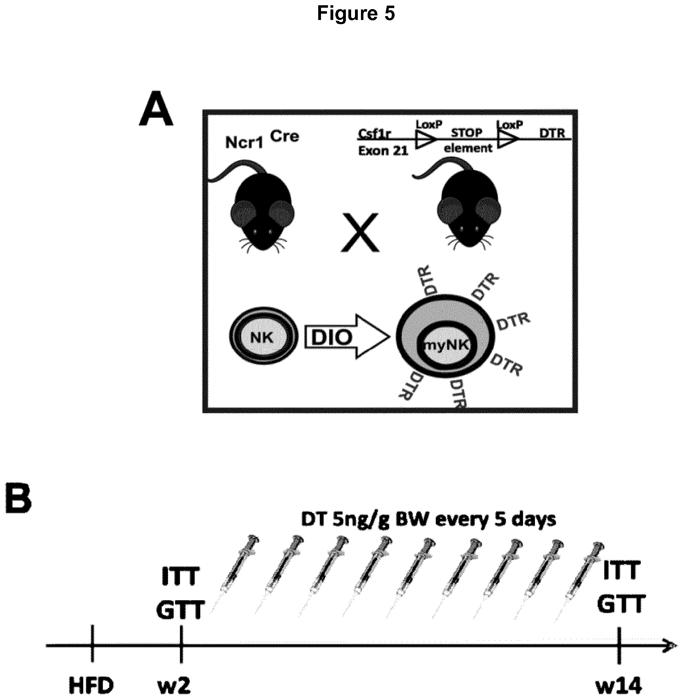

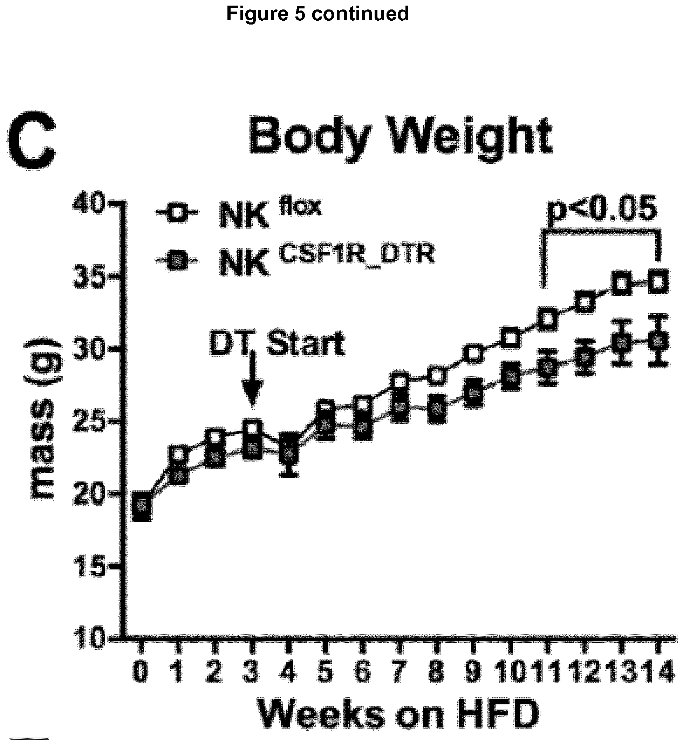

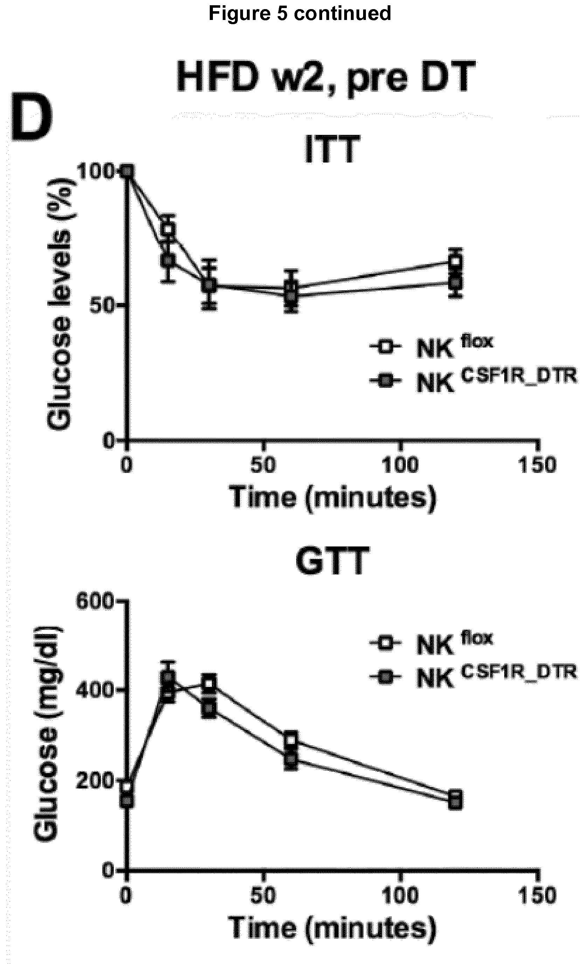

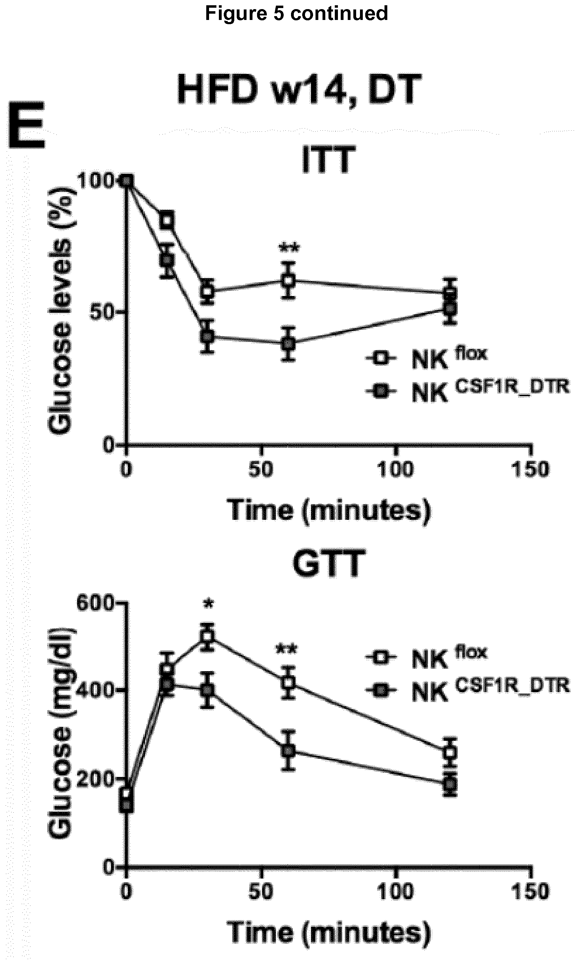

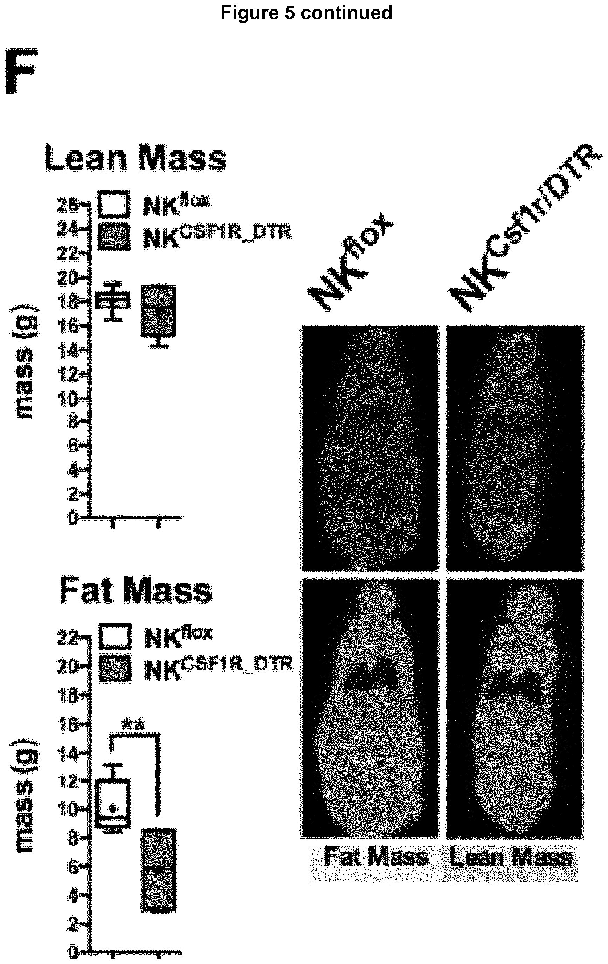

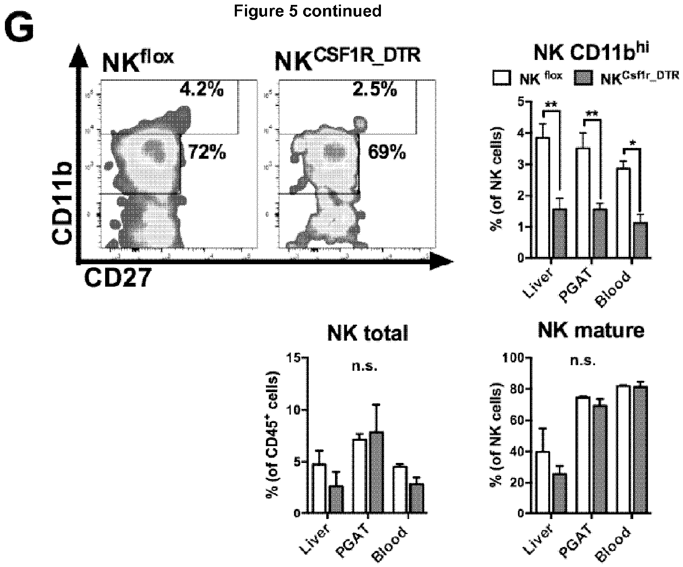

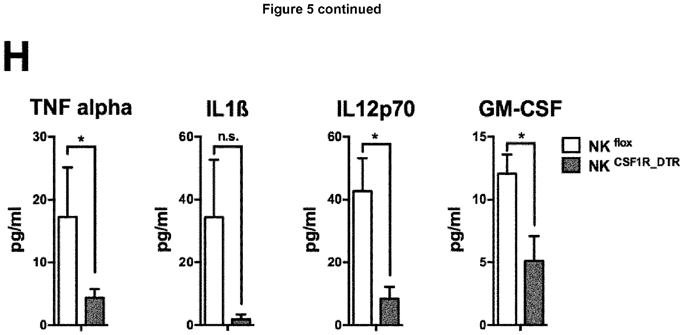

**Fig. 5****: Depletion of CD11b^{hi} NK-cells reduces obesity and improves metabolism in HFD fed mice**
   A) Breeding scheme to generate transgenic NK^{Csf1r_DTR} mice, in which during diet-induced obesity (DIO) the human diphtheria toxin receptor (DTR) is expressed in myeloid-gene expressing NK (myNK) cells under the control of the Csf1 r gene promotor.
   B) Experimental layout of longitudinal depletion of myNK-cells via intraperitoneal diphtheria toxin (DT) injections at a dose of 5ng/g body weight (BW) every 5 days.
   C) Body weight curves of NK^{Csf1r_DTR} (n=7) and NK^{flox} littermate control (n=8) mice subjected to repetitive DT injections starting two weeks after the beginning of HFD-feeding (statistics: 2-way ANOVA with Sidak's multiple comparison test).
   D) After two weeks of HFD-feeding but before DT injections NK^{Csf1r_DTR} (n=7) and NK^{flox} littermate control (n=8) mice were analyzed by insulin tolerance tests (ITT) and glucose tolerance tests (GTT).
   E) ITT and GTT analyses were repeated at end of the study in the same cohort of mice which then had received repetitive DT-injections (statistics: 2-way ANOVA with Sidak's multiple comparison test, *p ≤0.05, **p ≤0.01).
   F) Body composition of lean and fat masses determined by computed tomography was analyzed at the end of the 14-week HFD-feeding period in DT-injected NK^{Csf1r_DTR} (n=7) and NK^{flox} littermate control (n=8) mice.
   G) Flow cytometry analyses of NK-cells and CD11b expressing subpopulations from liver, PGAT and blood in DT-injected NK^{Csf1r_DTR} and NK^{flox} mice at the end of the DT-mediated depletion experiment (statistics: 2-way ANOVA with Sidak's multiple comparison test, *p ≤0.05, **p ≤0.01; n.s. not significant). H) Cytokine levels were determined in circulation from DT-injected NK^{Csf1r_DTR} and NK^{flox} mice at the end of the experiment (statistics: unpaired, two-sided t-test, *p ≤0.05).
   **H)** Cytokine levels were determined in circulation from DT-injected NKCsf1r_DTR 632 and NKflox mice at the end of the experiment (statistics: unpaired, two-sided t-test, *p ≤0.05).

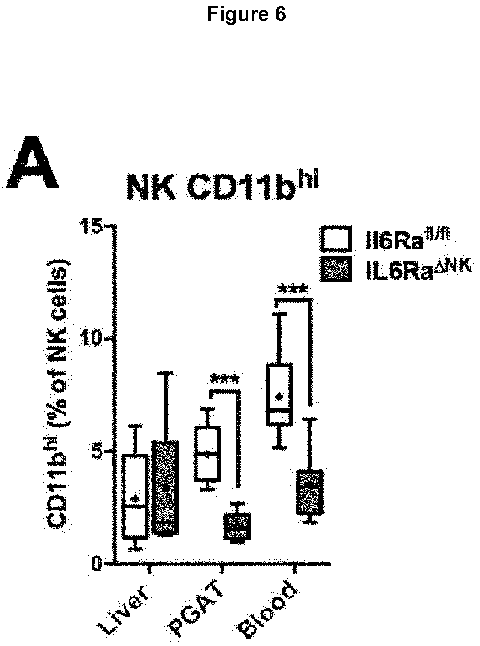

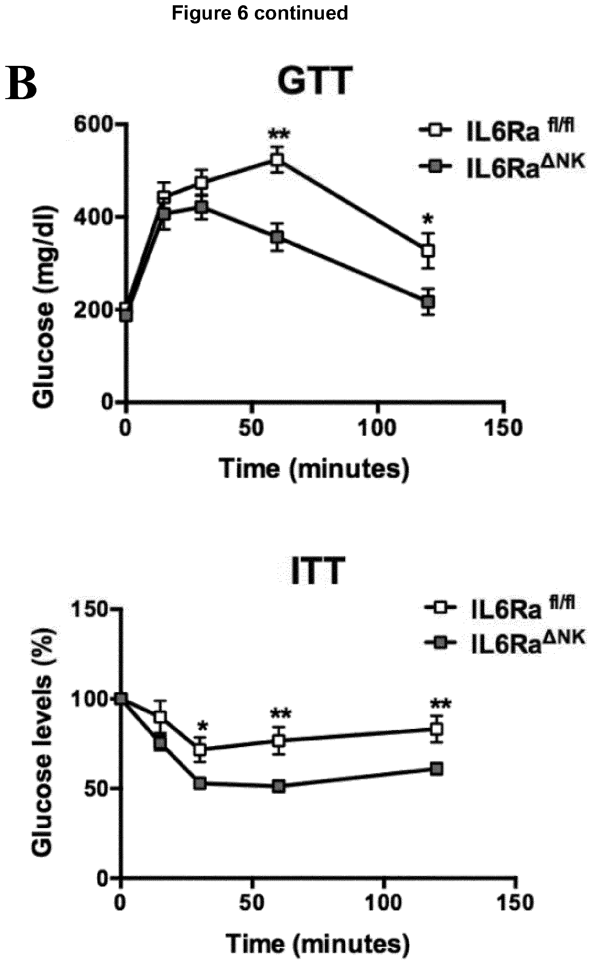

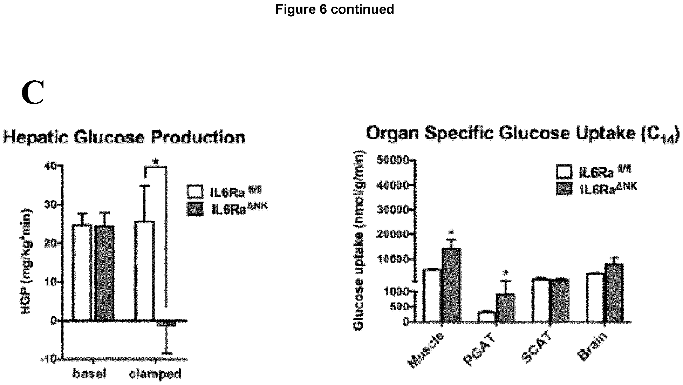

**Fig. 6****: Abrogation of IL-6R-signaling from NK-cells of mice prevents diet-induced obesity**
   **A)** Quantification of CD11b^{hi} NK-cells (gated on CD3-NK1.1⁺ NK-cells) in liver, PGAT and blood of IL6Ra^{fl/fl} (n=9) and IL6Ra^{ΔNK} (n=11) mice after 16 weeks of HFD feeding (statistics: 2-way ANOVA with Sidak's multiple comparison test; *p ≤0.05; n.s. not significant).
   **B)** Glucose (GTT) and insulin (ITT) tolerance tests were performed in IL6Ra^{fl/fl} (n=7) and IL6Ra^{ΔNK} (n=8) mice after 16 weeks of HFD-feeding (statistics: 2-way ANOVA with Sidak's multiple comparison test; *p ≤0.05, **p ≤0.01).
   **C)** Hepatic glucose production at the basal and clamped state and organ specific glucose uptake rates were determined in the same cohort of mice.

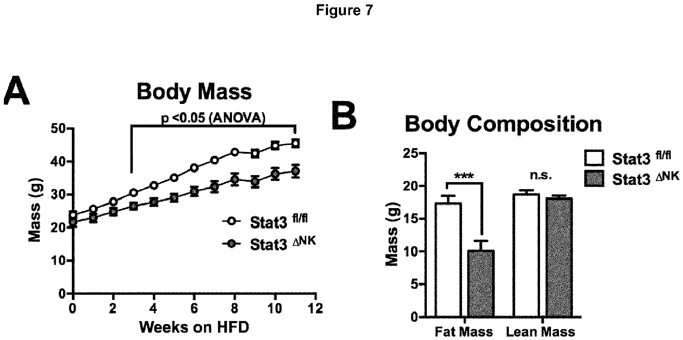

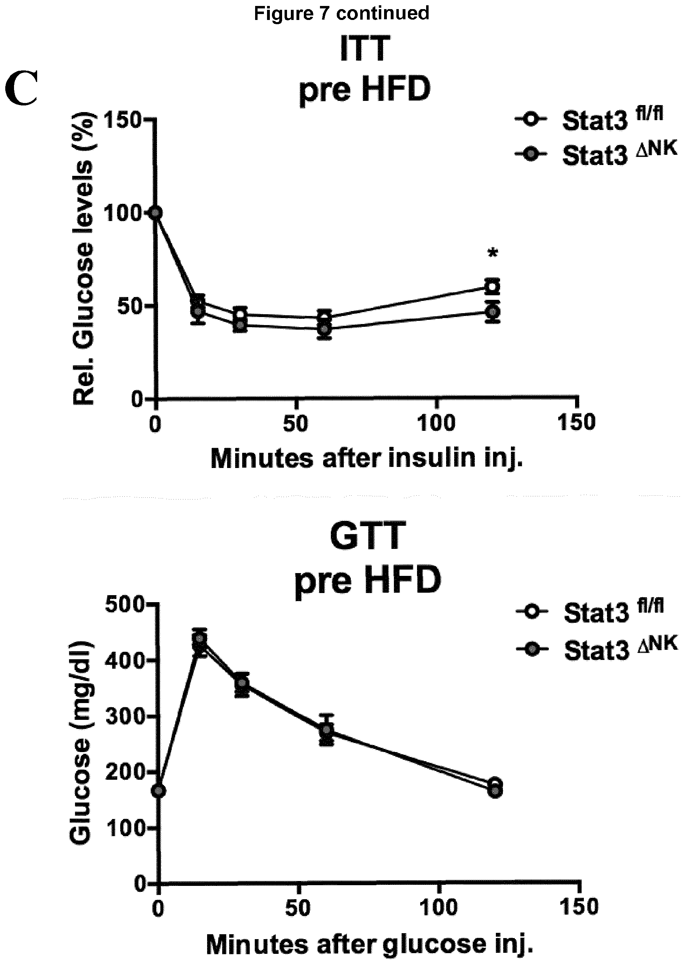

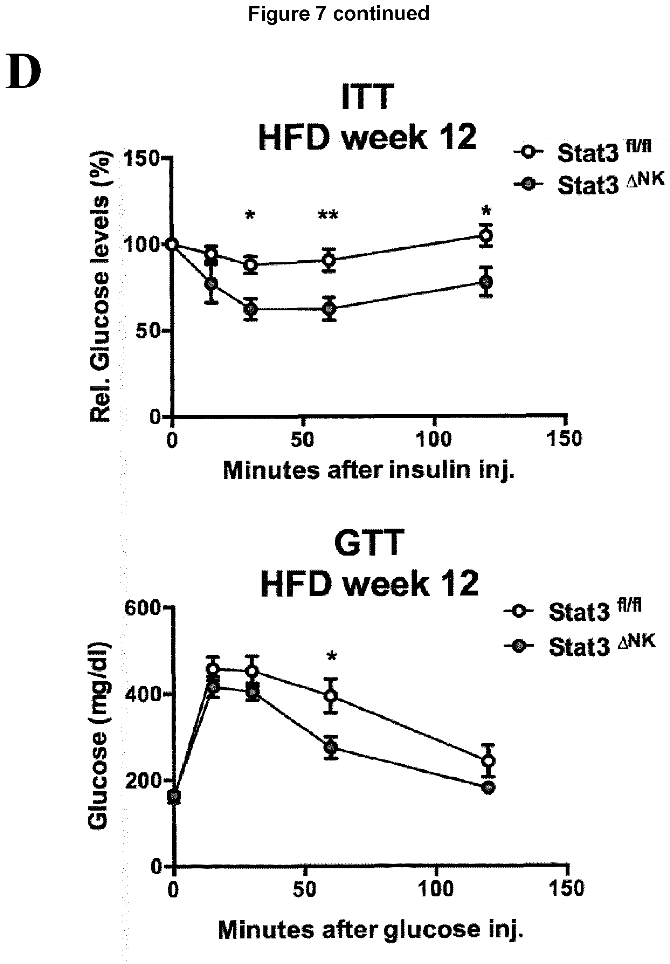

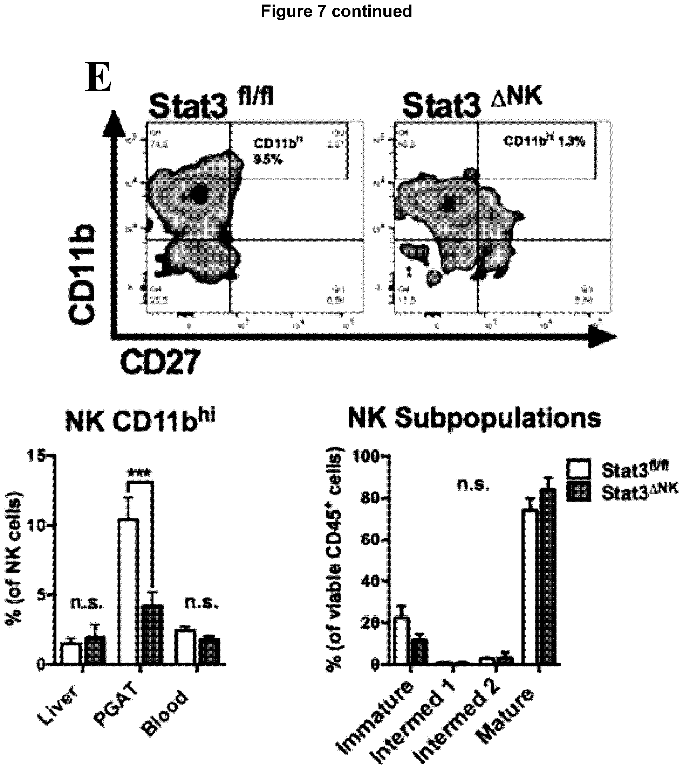

**Fig. 7****: NK-cell-specific disruption of Stat3 protects from obesity and CD11b^{hi} NK-cell formation**
   **A)** Body weights were assessed over a period of 12 weeks in HFD-fed mice lacking Stat3 expression in NK-cells (Stat3 ^{Δ NK}) and littermate controls (Stat3^{fl/fl}) (n=9vs9) (statistics: 2-way ANOVA with Sidak's multiple comparison test).
   **B)** Body composition after 12 weeks of HFD-feeding was analyzed in Stat3^{fl/fl} (n=9) and Stat3^{ΔNK} (n=9) mice by CT scans (statistics: unpaired, two-sided t-test, *p ≤0.05).
   **C+D)** Glucose (GTT) and insulin (ITT) tolerance tests were performed in Stat3^{fl/fl} and Stat3^{ΔNK} mice (n=7vs6) **E)** prior to HFD-feeding and **F)** after 12 weeks of HFD-feeding (statistics: 2-way ANOVA with Sidak's multiple comparison test; *p ≤0.05, **p ≤0.01).
   **E)** Representative dot blots of flow cytometry CD11 b/CD27 expression analysis in PGAT derived NK-cells after 12 weeks of HFD-feeding in Stat3^{fl/fl} and Stat3 ^{ΔNK} mice. Quantification of CD11b^{hi} NK-cells in liver, PGAT and blood of Stat3^{fl/fl} and Stat3^{ΔNK} mice (n=3vs4) and of the classic NK-cell subpopulations based on CD11 b/CD27 expression after 12 weeks of HFD-feeding (statistics: 2-way ANOVA with Sidak's multiple comparison test; *p ≤0.05; n.s. not significant).

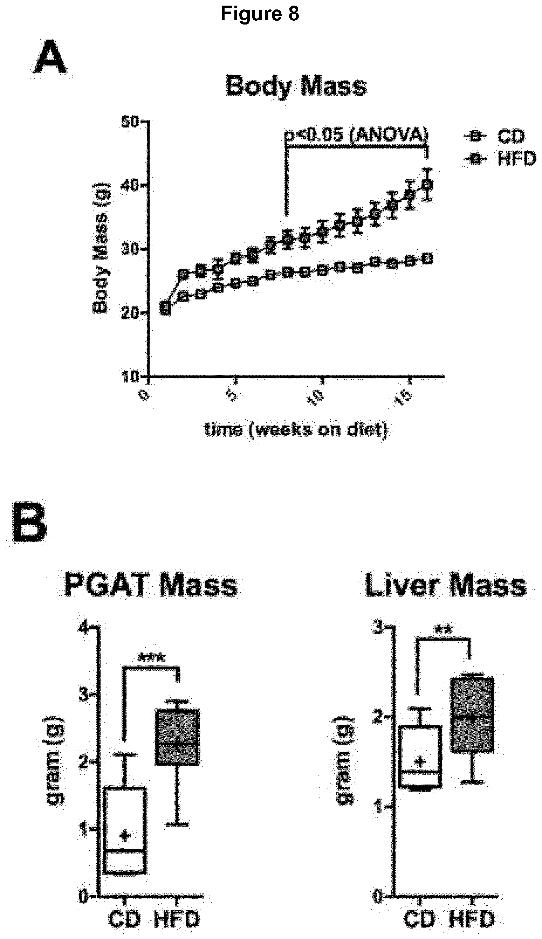

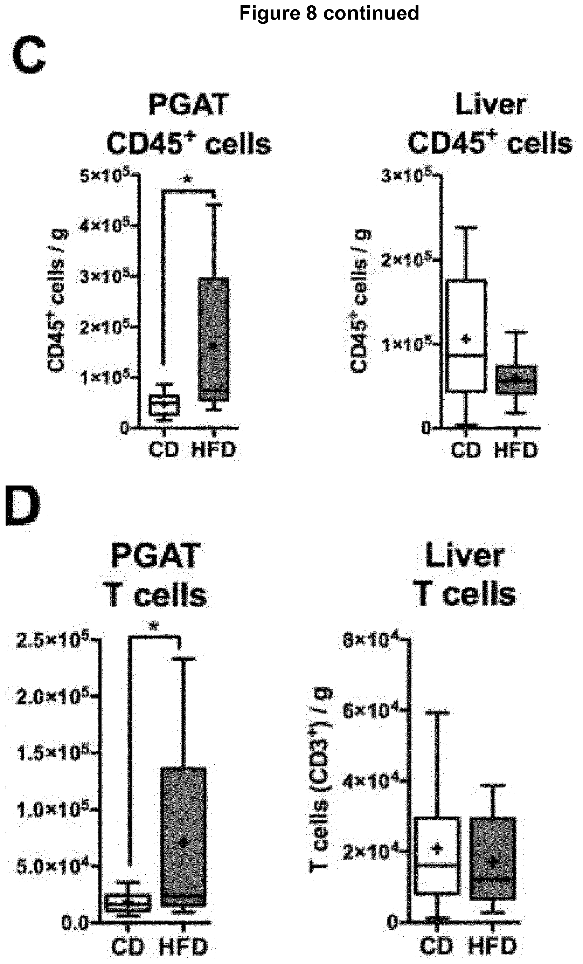

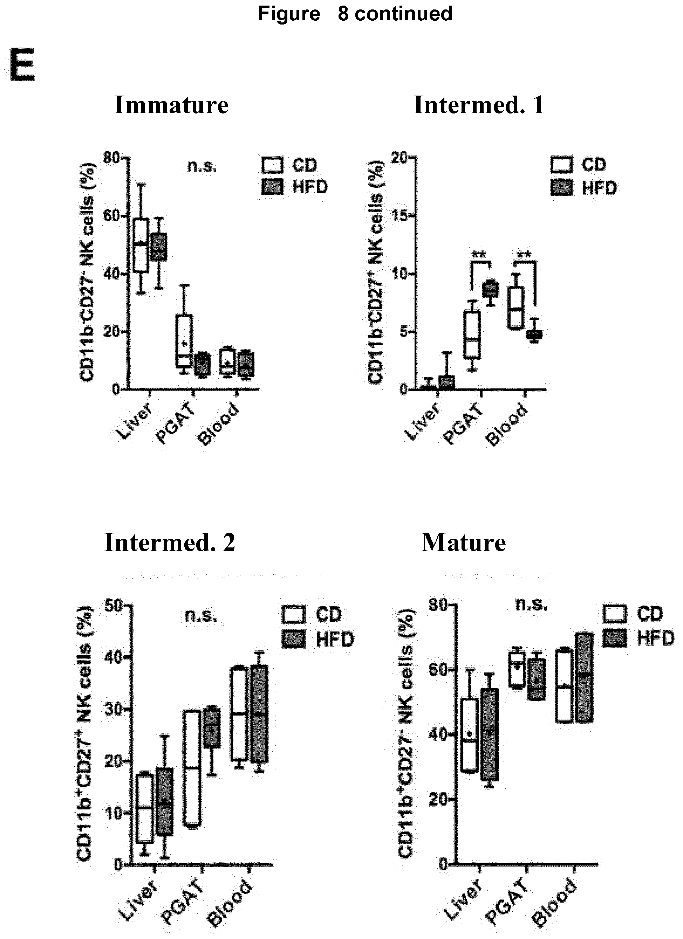

**Fig. 8****. Analysis of immune cell infiltrates in obese C57BL/6 wildtype mice**
   **A)** Body weight analysis weekly assessed in BI/6 wildtype mice during 16 weeks of HFD- or CD-feeding (n=18vs18) (statistics: 2-way ANOVA with Sidak's multiple comparison test).
   **B)** Organ mass analyses of PGAT and livers in these animals (n=18vs18) after 16 weeks diet (statistics: unpaired, 2-sided t-test; **p ≤0.01, ***p ≤0.001).
   **C+D)** Immune cell infiltration of **C)** CD45⁺ leucocytes and **D)** CD3⁺ T cells in PGAT and livers quantified per gram of tissue weight (statistics: unpaired, 2-sided t-test; *p ≤0.05).
   **E)** Distribution of NK-cell maturation states determined by CD11b/CD27 expression (CD11b⁻CD27⁻ immature, CD11b⁻CD27⁺ intermediate 1, CD11b⁺CD27⁺ intermediate 2 and CD11b⁺CD27⁻ mature) and
**Fig. 9****. Comparison of differentially regulated genes in CD11b^{hi} NK-cell to gene sets of innate lymphoid cell populations**
   A total of 1137 genes have been identified to be specifically regulated in CD11b^{hi} NK-cells compared to mature CD11b⁺ NK-cells. Figure 9 shows a table containing most important, significantly regulated genes (cut-off p≤0.05; fold-change ≥2).
**Fig. 10****. Expression analyses of Ncr1-Cre recombinase in NK, T and B cells** To examine the immune cell subpopulations in which the Ncr1^{Cre}-transgene is expressed, a fluorescent reporter mouse strain, tdTomato^{loxSTOPlox} mice (stock no. 007905, Jackson Lab), was crossed with Ncr1Cre mice leading to tdTomato-fluorescence upon cre-mediated recombination. **A)** Representative contour flow cytometry plots of tdTomato- expression in PBMC of Ncr1^{Cre+}tdTomato^{loxSTOPlox} mice further stained for NK-cells (NK1.1 and Ncr1), T cells (CD3) and B cells (CD19).
**Fig. 11****. Abrogation of IL6Ra signaling in NK-cells attenuates obesity in HFD-fed mice**
   **A)** Body weight development was assessed weekly over a period of 16 weeks in transgenic mice lacking IL6Ra expression in NK-cells (IL6Ra ^{Δ NK}) and littermate control mice (IL6Ra^{fl/fl}), which were either subjected to high-fat-diet (HFD) (IL6Ra^{fl/fl} n=10; IL6Ra^{ΔNK} n=13) or control diet (CD) (IL6Ra^{fl/fl} n=10; IL6Ra ^{Δ NK} n=6) feeding (statistics: 2-way ANOVA with Sidak's multiple comparison test).
   **B)** Organ masses after 16 weeks of HFD-feeding (IL6Ra^{fl/fl} n=10; IL6Ra^{ΔNK} n=13).
   **C)** Body fat content of IL6Ra^{fl/fl} (n=10) and IL6Ra^{ΔNK} (n=8) after 16 weeks of HFD was determined by NMR analysis (statistics: unpaired, two-sided t-test, *p ≤ 0.05).

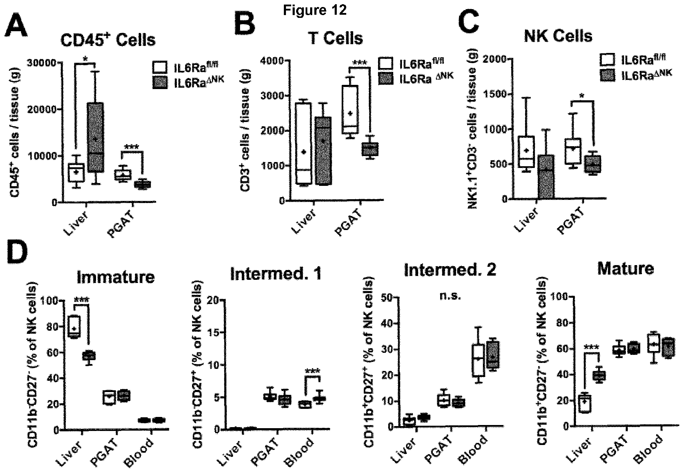

**Fig. 12****. NK-cell specific knockout of the IL6Ra attenuates metaflammation in PGAT** To assess the level of metaflammation, the levels of cellular infiltration were analyzed by FACS of
   **A)** CD45⁺ leucocytes, **B)** CD3⁺ T cells and **C)** CD3⁻NK1.1⁺ NK-cells in liver and PGAT of IL6Rafl/fl (n=9) and IL6Ra^{ΔNK} (n=11) mice after 16 weeks of HFD-feeding. Given numbers are cells per tissue mass. (Statistics: unpaired, two-sided t-test; *p ≤0.05, **p ≤0.01, ***p ≤0.001)
   **D)** NK-cell maturation states were analyzed in these mice by CD11b/CD27 staining and flow cytometry. (Statistics: 2-way ANOVA with Sidak's multiple comparison test; n.s. not significant, *p ≤0.05, **p ≤0.01, ***p ≤0.001)

### EXAMPLES

### Material and Methods

### Animal care and generation of transgenic mouse strains

All mouse experiments were approved by the local authorities (Bezirksregierung Köln; Germany) and conducted in accordance with NIH guidelines. Mice were housed in groups of 3-5 animals at 22-24°C and a 12-hour light / dark cycle. Animals had ad libitum access to food and water at all times, and food was only withdrawn if required for an experiment. In general, experiments started with 6-week old animals.

C57BI/6JR wild-type mice were purchased from Janvier (Janvier Labs, France) at the age of 4 weeks and acclimatized to our facility over two weeks prior to the start of experiments. NK-cell specific transgenic mouse models, used in this study, were generated by crossing previously published mouse strains all of which have been backcrossed to C57BL/6 mice for at least ten generations. Briefly, mice with a conditional knockout of the IL6Ra gene in NK-cells (IL6Ra ^{Δ NK} mice) were generated by crossing Ncr1^{Cre} mice (kindly provided by Dr. Emilio Casanova, Ludwig Boltzman Institute for Cancer Research, Vienna, Austria) with IL6Ra-floxed mice and line breeding was maintained with Ncr1^{Cre+/-} IL6Ra^{fl/fl} crossed with Ncr1^{Cre-/-} IL6Ra^{fl/fl} mice.

Mice in which NK-cells are specifically enabled to express DTR upon activation of the Csf1r-gene (NK^{Csf1r/DTR} mice), a myeloid marker gene, were generated by crossing Ncr1^{Cre+/-} mice with heterozygous Csf1r^{LoxStopLox-DTR} mice (3) (kindly provided by Dr. Ana Domingos, Instituto Gulbenkian de Ciência, Oeiras, Portugal). An NK-cell specific knockout of the Stat3 gene was generated by crossing Ncr1^{Cre+/-} mice with Stat3-floxed mice and line breeding was maintained with Ncr1^{Cre+/-} Stat3^{fl/fl} crossed with Ncr1^{Cre-/-} Stat3^{fl/fl} mice.

### Diet induced obesity, high fat diet feeding

For all feeding experiments the same conditions were used. Animals at the age of six weeks were either fed a high-fat diet (HFD) containing 60% calories from fat, 21% calories from carbohydrates and 19% calories from protein (ssniff D12492 (I) mod., ssniff Spezialdiäten GmbH, Germany) or a low-fat control diet containing 13% calories from fat, 67% calories from carbohydrates and 20% calories from protein (ssniff D12450B mod. LS, ssniff Spezialdiäten GmbH).

### Diphtheria toxin mediated cell ablation

In order to deplete myeloid (CD11b^{hi}) NK-cells, intra-peritoneal injections of diphtheria toxin (Sigma Aldrich) at a dose of 5ng per gram body weight every five days were given to NK^{Csf1r/DTR}- mice and littermate controls.

### Human samples

After obtaining informed consent, anthropometric data were prospectively assessed and collected blood samples from obese and lean individuals and cryopreserved peripheral blood mononuclear cells (PBMCs) immediately after Ficoll density gradient centrifugation and washing steps.

Plasma samples were cryopreserved in liquid nitrogen immediately after centrifugation (15,000xg, 10 minutes). The study (NK-ADIPO) was approved by the institutional review board (No. 15-042, Medical Faculty, University of Cologne, Cologne, Germany).

### Immune cell isolation and flow cytometry

Leucocytes were isolated from adipose tissue, liver and peripheral blood according to modified protocols published elsewhere (Miltenyi Biotech). Briefly, organs were dissociated with a tissuelyser (GentleMACS, Miltenyi) and digested enzymatically for 20 min at 37°C while continuous shaking: adipose tissue: type I collagenase 500U/ml; DNAse1 150U/ml. liver: type IV collagenase 500U/ml; DNAse1 150U/ml (all from Worthington, Lakewood, USA). Immune cells were separated from stromal cells by centrifugation: adipose tissue homogenates 400xg for 5 minutes and liver homogenates via 20%-histodenz (Sigma Aldrich) density-gradientcentrifugation. Leucocytes from blood samples were generated by standard erythrocyte lysis in ammonium chloride solution (eBioscience) for 5-10 minutes. Finally, cell suspensions were resuspended in FACS buffer (MACS-Buffer, Miltenyi Biotech) and passed through a 40 *µ*m strainer (BD Biosciences) to remove large cellular debris.

For flow cytometry analysis, mouse (m) or human (h) leucocytes were stained after FC-blocking with either anti-mouse CD16/32 or human-IgG (Trustain, Biolegend) followed by fixable dead cell staining (LIVE/DEAD, Invitrogen). Directly fluorochrome-conjugated anti-mouse or anti-human antibodies or the respective isotype control were used for specific immunostainings (1:50-100 dilutions, all from Biolegend, San Diego, if not otherwise indicated):
mCD45-BV510 (30F11), mCD3-PacificBlue (17A2), mNK1.1-A700 (PK136), m/hCD27-PerCP-Cy5.5 or -PE-Cy7 (LG.3A10), m/hCD11b-PE-TexR (M1/70.15; Invitrogen) or m/hCD11b-APC or -APC-Cy7 (M1/70), mCD25-PE (7D4; Miltenyi Biotech), mCD69-PE-Cy7 (H1.2F3), mCCR2-FITC (FAB5538F, R&D Systems), mNKG2D-PE (CX5), mNKG2A-APC (16A11), mLy49D-AF647 (4E5), mLy49H-PE (3D10), mLy49C/I-Fitc (5E6; BD Pharmigen), mNKp46-Fitc or -PE or -BV421 (29A1.4), mKLRG1-PE-Cy7 (2F1/KLRG1), mCsf1r-PE or -Fitc (AFS98), mIL6RaPE or -APC (D7715A7), hCD3-PacificBlue (OKT3), hCD16-Fitc (3G8), hCD56-PercP-Cy5.5 (HCD56), hIL6Ra-APC (UV4).

Cells were analyzed using an 8-color flow cytometer (MACSquant-10, Miltenyi Biotec) or a 10-color flow cytometer (Gallios, Beckman Coulter) and respective data analysis was performed with FlowJo (Treestar) or Kaluza (Beckman Coulter) software packages in the recent versions. In all analyses, the first gate identified lymphocytes by forward and sideward scatter followed by exclusion of doublets. Analysis of NK-cells, defined as CD3- NK1.1⁺ and/or Ncr1⁺ (for murine cells) and CD3-CD19-CD14- CD16⁺ and/or CD56⁺ (for human cells), was always based on viable (dead stain negative) CD45⁺ cells.

### Cell sorting

NK-cell subpopulations from murine or human samples were purified from single cell suspensions using FACSAria-III or FACSAria-Fusion cell sorters (BD Bioscience) after immunostainings as described above. To sort mouse CD11b^{hi} and CD11b⁺ mature NK-cells from organs and blood, gates were set on single, viable CD45⁺CD3⁻NK1.1⁺ lymphocytes. To sort human CD11b^{hi}IL6Ra⁺ versus CD11b⁺IL6Ra⁻ NK-cells from blood, gates were set on single, viable CD3-cells single or double positive for CD16 and CD56. Purified cells were directly sorted into RNA-protect cell reagent (Qiagen, Germany).

### RNA isolation from purified cells and mRNA sequencing

Stabilized, purified NK-cell populations were pelleted by centrifugation and total RNA was extracted using the Arcturus RNA picopure Kit (KIT0204, ThermoFisher Scientific) following the manufacturer's instructions. RNA integrity was assessed with the Agilent 2100 Bioanalyzer.

RNA libraries were prepared from a minimum of 100ng total RNA using the TruSeq® RNA sample preparation Kit v2 (illumina). Complementary DNA (cDNA) was transcribed from poly-A selected RNA, which served for library generation. Libraries were sequenced in replicates for 30 million reads on an Illumina HiSeq 2000 sequencer with a paired- end (101x7x101 cycles) protocol.

### Single Cell RNA-seq analysis

Read 2 of the read pair was first 3' trimmed for adapters, base quality and poly-A tails using cutadapt 1.9.1 (http://dx.doi.org/10.14806/ej.17.1.200). Remaining reads were mapped to the mouse genome GRCm38 (primary assembly) using STAR-2.5.2b (https://www.ncbi.nlm.nih.gov/pubmed/23104886). Gene models were used according to gencode version M9 (https://www.ncbi.nlm.nih.gov/pubmed/26187010). Gene summarization was done using feature Counts 1.5.0-p1 (Liao et al., 2014) collapsing exons to genes and excluding pseudogenes and transcripts with a biotype related to "decay". Multimapping reads were discarded. Cell and gene demultiplexing was done using the cell-barcode and the unique molecular identifier (UMI) present in the first 12nt of Read 1 of the read pair.

Data analysis was performed using RaceID2 and StemID algorithm (Grün et al., 2016). Downsampling to 800 transcripts was used for data normalization. K-medoids clustering was performed using log-pearson correlation as a distance metric. The minimum suitable cluster number (=3) characterizing the dataset was determined by computing Jaccard's similarity for each cluster by bootstrapping for k-medoids clustering with different cluster numbers. The minimum number yielding a Jaccard's similarity >0.6 for all clusters was selected. The t-distributed stochastic neighbor embedding (t-SNE) algorithm was used for dimensional reduction and cell cluster visualization (Maaten and Hinton, 2008). RaceID2 was executed with the probability threshold value for outlier identification set to <10 -3. The StemID algorithm was used to infer a dedifferentiation trajectory. A p-value threshold of 0.05 was chosen to assign significance to the links.

### Bioinformatic analysis

Gene expression analysis was performed at the CECAD bioinformatic core facility (Dr. Peter Frommolt, University of Cologne, Germany) using the publically available QuickNGS software platform (http://athen.cecad.uni-koeln.de/quickngs/web) which integrates well-established algorithms (Tophat2, Cufflinks2, DESeq2 and DEXSeq). Heatmaps and unsupervised cluster analyses were generated from log2-transformed FPKM values. After filtering the genes according to the variance of their FPKM values across the samples, only the 1000 genes with highest variance were displayed.

Gene set enrichment and gene ontology analyses of were performed with publically available programs (http://geneontology.org/) and the Ingenuity software package (Qiagen, Germany). Statistical analysis of enriched gene ontologies was corrected for multiple testing and only significant results (p ≤0.05) were considered. Analyses were performed with The Ingenuity software package was also employed to align the CD11b^{hi} NK-cell gene signature to those of different ILC populations and B220⁺ pre-mNK-cells derived from the Gene Expression Omnibus Repository (http://www.ncbi.nlm.nih.gov/geo/). The immunological genome project data base (https://www.immgen.org/) enabled us to align the CD11b^{hi} NK-cell specific gene set to a large panel of immune cell specific gene sets.

### Analysis of plasma samples in mice and humans

*Mouse samples:* Insulin plasma concentrations were determined by ELISA with mouse standards according to the manufacturer's guidelines (mouse high-sensitivity Insulin ELISA, Crystal Chem, USA). Blood glucose levels were determined from tail vein blood using an automatic glucometer (Bayer Contour, Bayer, Germany). Cytokines (TNF-alpha, IL-1beta, IL-12p70 and GM-CSF) were detected in replicates of undiluted 50 µl plasma samples using a multiplex magnetic bead immunoassay (Life Technologies) and quantification was performed on a Bio-Plex 200 reader (BioRad) according to the manufacturer's instructions.

*Human samples:* Analyses of insulin, glucose and hsCRP plasma concentrations were performed in the central clinical laboratory of the University Hospital Cologne (Cologne, Germany). HOMA-IR was calculated as previously described [glucose (mg/dl) x insulin (mU/l)/ 405].

### Glucose and insulin tolerance tests

Glucose tolerance tests (GTT) were performed with 6h-fasted mice at the indicated age and time of diet-feeding given for each experiment. Blood glucose concentrations (mg/dl) were measured following fasting, prior to the test, and 15, 30, 60 and 120 minutes after intraperitoneal injection of glucose 20% (1.5mg/g BW) (DeltaSelect). Blood glucose levels were determined from tail vein blood using an automatic glucometer (Bayer Contour, Bayer, Germany).

Insulin tolerance tests (ITT) were performed with 2h-fasted mice at corresponding time points to GTTs. Blood glucose concentrations (mg/dl) were measured following fasting, prior to the test, and 15, 30, 60 and 120 minutes after intraperitoneal injection of insulin (0.75mU/g BW, Insuman rapid, Sanofi Aventis).

### Hyperinsulinemic-euglycemic clamp studies in awake mice

Surgical implantation of catheters into the jugular vein was performed as described. After 5-6 days of recovery, only mice that had lost less than 10% of their preoperative weight were included. Each animal was deprived of food for 4 h in the morning of the experiment. All infusions used in the experiment were prepared with a 3% plasma solution obtained from fasted donor mice. A primed-continuous infusion of tracer d-[3-3H]-glucose was initiated 50 min before the clamping (5 µCi priming at a rate of 0.05 µCi/min; PerkinElmer). After a 50-minute basal period, a blood sample was collected from the tail tip for determination of basal parameters. The clamping began with a primed-continuous insulin (INSUMAN rapid; Sanofi-Aventis) infusion (40 µU prime per gram body weight, followed by a continuous rate of 4 µU per g body weight per min) and glucose concentrations in blood were measured every 10 min (Bglucose analyzer, Hemocue). Euglycemic serum levels (120-140 mg/dl) were maintained by adjustment of a 20% glucose infusion (DeltaSelect). Approximately 120 min before the end of the experiment, 2-[1-14C]-deoxy-d-glucose (10Ci; American Radiolabeled Chemicals) was infused, and blood samples were collected until steady state was reached. The steady state was considered, when a fixed glucose-infusion rate maintained the glucose concentration in blood constant for 30 min. During the steady state, blood samples were collected for the measurement of steady-state parameters.

At the end of the experiment, mice were killed by cervical dislocation, and brain, liver, PGAT, SCAT and skeletal muscle were dissected, snap-frozen in liquid nitrogen and stored at -80°C. The [3-3H] glucose radioactivity of plasma in basal conditions and at steady state was measured as described. The radioactivity of 2-[1-14C]-deoxy-d-glucose in plasma was measured directly in a liquid scintillation counter. Lysates of adipose tissue and skeletal muscle were processed through ion-exchange chromatography columns (AGR1-X8 formate resin, 200-400 mesh dry; Poly-Prep Prefilled Chromatography Columns; Bio Rad Laboratories) for the separation of 2-[1-14C]-deoxy-d-glucose from 2-[1-14C]-deoxy-d-glucose-6-phosphate, 2-[1-14C] (2DG6P).

The glucose-turnover rate (mg × kg⁻¹ × min⁻¹) was calculated as described (Konner et al., 2007). The uptake of glucose in brain, PGAT, SCAT and skeletal muscle in vivo (nmol ×g⁻¹ × min⁻¹) was calculated on the basis of the accumulation of 2-[1-14C]-deoxy-d-glucose-6-phosphate, 2-[1-14C] in the respective tissue and the disappearance rate of 2-[1-14C]-deoxy-dglucose from plasma, as described.

### Body composition analysis

Body weights were assessed weekly. Lean and fat mass were determined using the NMR Analyzer Minispeq mq7.5 (Bruker Optik). Alternatively, body composition was analyzed by computed tomography (CT) in isoflurane-anesthetized mice (Dräger and Piramal Healthcare).

For data acquisition on an IVIS Spectrum CT scanner (Caliper LifeScience, USA) IVIS LivingImage Software V4.3.1 was used. Quantification of lean and fat mass contents were determined with a modification of the previously described Vinci software package 4.61.0 developed at our institution.

### Indirect Calorimetry

Indirect calorimetry was performed using an open-circuit, indirect calorimetry system (PhenoMaster, TSE systems). Mice were trained for three days before data acquisition to adapt to the food/drink dispenser of the PhenoMaster system. Afterwards mice were placed in regular type II cages with sealed lids at room temperature (22°C) and allowed to adapt to the chambers for at least 24 hours. Food and water were provided ad libitum. All parameters were measured continuously and simultaneously.

### Immunoblots

Protein extraction from cryopreserved tissues and SDS-page immunoblot procedures were done as previously described. Membranes were blocked with 5% WB-blocking reagent (Roche, Switzerland) in Tris-buffered saline containing 0.2% Tween-20 (TBS-T), and incubated with primary antibodies at 4°C overnight. The following primary antibodies and dilutions were used: anti-phosphoAKT^{Ser473} (1:1000, #4060, Cell Signaling), anti-panAKT1-3 (1:1000, #9272, Cell Signaling) and, as a loading control, anti-calnexin (1:2000, Cat 208880, Merck Milipore). Quantification of chemiluminescent signals was performed with the ImageJ (Fiji)-software package.

### JNK kinase assay

Analysis of c-jun N-terminal kinase (JNK) activity in white adipose tissue and liver protein lysates was performed with the non-radioactive JNK kinase assays kit (#8794, Cell Signaling) according to the manufacturer's instructions. Briefly, phosphorylated JNK (p-JNK) was immunoprecipitated from tissue lysates with rabbit anti-mouse-p-JNK^{Thr183,Tyr185} (#8794; Cell Signaling) coupled to Sepharose beads. *In vitro* phosphorylation of recombinant c-Jun protein as a JNK-substrate was performed in ATP containing kinase buffer. Finally, the amount of phosphorylated c-Jun^{Ser63}, as a measure of JNK-activity, was determined by immunoblot analysis. Probes of the initial lysates before immunoprecipitation were used as loading controls.

### Histological analyses

Liver and adipose tissues were harvested following mouse scarification, fixed in paraformaldehyde 4% and embedded in paraffin. Hematoxylin and eosin (HE) staining of tissue sections (4-5µm) were performed according to standard procedures. F4/80 immunohistochemical staining was performed with 1:100 diluted primary anti-F4/80 antibody (MCA497G, Serotec) as previously described. F4/80 positive crown-like structures were quantified with a LeicaDM1000 LED microscope (Leica, Germany). Quantitative image analyses were performed with the ImageJ (Fiji) software package (NIH) including the Adiposoft plugin (http://imagej.net/Adiposoft).

### Statistical Analyses

All data, unless otherwise indicated, are shown as mean values ± standard error of the mean (SEM). In box-and-whisker plots the upper and lower whiskers indicate the minimum and maximum values of the data, centerlines indicate the median, and the mean is represented by a plus sign. Comparison of two independent groups was performed with unpaired two-tailed Student's t-test. Data sets with more than two groups were analyzed using one-way analysis of variance (ANOVA) followed by Tukey's posthoc test. For statistical analyses of longitudinal data, i.e. body weight curves, GTTs, ITTs and clamp studies (glucose infusion rate), two-way ANOVA was performed corrected by Sidak's multiple comparison test. All figures and statistical analyses were generated using the GraphPad Prism 6 software. P-values ≤ 0.05 were considered statistical significant.

### Example 1: Obesity promotes formation of Myeloid NK-cells in mice

In light of the recently described role of NK-cells in the pathophysiology of obesity and obesity-associated insulin resistance, the inventors analyzed NK-cell development and tissue distribution during the course of obesity development in mice. Mice, which had been fed either a normal chow (CD) or a high-fat diet (HFD) from the age of 6 weeks on, were analyzed. As expected, mice exposed to HFD exhibited a significant, 140% increase in body weight, an almost threefold increase in perigonadal adipose tissue (PGAT) mass and significantly increased liver weight compared to CD-fed animals (Fig. 7A, B). In parallel to progressive obesity during 16 weeks of HFD-feeding, tissue mass related total numbers of CD45⁺ immune cells increased in PGAT, but not in liver (Fig. 7C). This was paralleled by increased T-cell numbers in PGAT, a fact previously reported (Fig. 7D). As observed for T cells, also the numbers of NK-cells significantly increased in PGAT of mice upon HFD-feeding (Fig. 1A).

To further investigate NK-cell maturation in obesity, multicolor flow cytometry analyzing CD11b/CD27 immunoreactivity in (CD45⁺NK1.1⁺CD3⁻) NK-cells as well established maturation markers were employed. Here, no consistent major alterations in the proportion of immature, intermediate 1, intermediate 2 and mature NK-cells in circulation or the investigated tissues were detected, neither early nor late during obesity development (Fig. 1B and Fig. 7E). However, in PGAT as well as in the circulation, HFD-feeding provoked the appearance of a previously unidentified NK-cell subpopulation characterized by higher levels of CD11b expression compared to CD11b⁺ mature NK-cells (Fig. 1B, C). In addition, increased NK-cell numbers expressing the activation markers CD25 and CD69 as well as the chemokine receptor CCR2, most pronounced in PGAT of HFD-fed mice (Fig. 1 D) were detected. In contrast, expression of the activatory and inhibitory NK-cell receptors NKGD2, NKG2A, Ly49D, Ly49H, Ly49C/I, NKp46 (Ncr1) and KLRG1 remained largely unaltered upon obesity development. This allows to conclude that during obesity development, NK-cells are an important immune cell population infiltrating PGAT. Most strikingly, obesity promoted the expansion of a distinct NK-cell population in circulation and PGAT characterized by high level expression of CD11b, referred to as CD11b^{hi} NK-cells.

### Example 2: Further Analysis of Murine CD11b^{hi} NK subpopulation, morphological analysis and expression profile

To further characterize this obesity-associated CD11b^{hi} NK-cell population, the inventors analyzed morphologic features of flow-sorted CD11b^{hi} and CD11b⁺ (mature) NK-cells (CD45⁺NK1.1⁺CD3⁻). CD11b^{hi} NK-cells were significantly larger and more granulated compared to mature NK-cells, irrespective whether they were isolated from PGAT or circulation (Fig. 2A). Furthermore, cytomorphological analysis of both NK-cell types confirmed the distinct large cell appearance of CD11b^{hi} NK-cells compared to their mature CD11b⁺ counterpart (Fig. 2A).

To further define CD11b^{hi} NK-cells at a molecular level, gene expression profiles of flow-sorted CD11b^{hi} and mature NK-cells isolated from circulation or PGAT were compared by total mRNA deep-sequencing. Unsupervised hierarchical cluster analyses revealed a trunk of similar gene expressions in all probes and a comparable gene expression pattern of mature NK-cells isolated from circulation or PGAT (Fig. 2B). Also, gene expression profiles of CD11b^{hi} NK-cells isolated from both compartments revealed a high degree of similarity (Fig. 2B). However, when gene expression between CD11b^{hi} NK and mature NK-cells was analyzed pronounced differences in gene expression signatures were uncovered, with 1675 and 2499 genes increased and 171 and 510 genes decreased in CD11b^{hi} and mature NK-cells respectively (cut-off: fold change 2; p-value 0.01). A shared signature of 1137 genes differentially expressed in CD11b^{hi} NK-cells from circulation and PGAT was found to be up-regulated compared to mature NK-cells (cut-off: fold change 2; p-value 0.01). This gene set was subjected to gene ontology analysis, which revealed a significant enrichment of gene sets in CD11b^{hi} NK-cells affecting cytokine-associated signaling, myeloid differentiation, NK-cell activation and chemotaxis (Fig. 2C).

Among the most differentially regulated genes were the IL-6 receptor alpha (IL6Ra) and the CSF-1 receptor (Csf1r) with a 10-fold upregulation for IL6Ra and 15-fold upregulation for Csf1r (Fig. 2C). Of note, both genes were previously reported to be either absent or expressed only at low levels in classical NK-cells. Their expression was thus validated in CD11b^{hi} NK-cells by flow cytometry. While only 9% of mature NK-cells express the IL6Ra, the proportion of IL6Ra⁺ cells was increased to 90% in CD11b^{hi} NK-cells. Similarly, the proportion of Csf1r-expressing NK-cells was significantly increased (3-fold) in the CD11b^{hi}-population.

The inventors next related the 1137 genes differentially expressed in CD11^{hi} NK-cells to other immune cells and aligned this gene signature to publically available gene expression profiles of B cells, T cells, dendritic cells (DCs), monocytes, macrophages, granulocytes and NK-cells. The highest degree of overlap was found with myeloid immune cells (i.e. DCs, monocytes, macrophages, B cells and granulocytes). The overlap with other cell types was less prominent. Of note, the CD11b^{hi} NK-cell gene signature shared only very limited overlap with gene expression signatures of defined innate lymphoid cell (ILC)-populations or pre-mNK-cells (formerly described as B220⁺ NK-cells or interferon producing killer dendritic cells, (IKDC)) (Fig. 8A). However, comparison of upstream regulatory pathways, which resulted from gene set enrichment analyses in CD11b^{hi} NK-cells and pre-mNK-cells, uncovered potential similarities of both cell types although pre-mNK-cells share only 4.3% of the differentially expressed genes found in CD11b^{hi} NK-cells (Fig. 8B).

Collectively these analyses allow to conclude that CD11b^{hi} NK-cells, which drastically increase in obesity, share highest morphological and molecular similarity with cells of the myeloid lineage, members of which have well-established functions in obesity associated insulin resistance. Therefore, the cells of the newly identified CD11b^{hi} NK-cell subpopulation is herein called myeloid NK-cells (myeloid NK-cells). The increased IL6Ra expression appears to be a discriminative marker for myeloid NK-cells compared to mature NK-cells.

### Example 3: Identification and Investigation of Myeloid NK in human subjects

Having identified increased myeloid NK-cells in PGAT and circulation of obese, insulin resistant mice, the inventors next investigated whether a similar NK-cell population is detectable in obese humans. To address this point, cohorts of lean and obese human subjects were recruited and the number and marker gene expression of NK-cells in circulation was analyzed. Analysis of NK-cell subsets based on CD56 and CD16 expression revealed no differences in circulation between lean and obese human subjects (Fig. 3A). However, when NK-cells were stained for CD11b and CD27 to further define NK-cell maturation states, a significant increase in the proportion of CD11b^{hi} NK-cells in the circulation of obese versus lean human subjects was observed (Fig. 3B). In line with the findings in mice, NK-cells from obese individuals displayed increased IL6Ra expression and the numbers of CD11b/IL6Ra double-positive NK-cells were significantly increased in the circulation of obese human subjects (Fig. 3C, D).

To further investigate a possible dynamic regulation of myeloid NK-cells during weight gain and loss in humans, blood samples from human subjects undergoing a controlled weight reduction program (Optifast) were prospectively collected. This program comprised of a 3-month phase of massive weight reduction due to caloric restriction and a weight maintenance phase of another 3 months. Blood samples were taken before (t0) and at the end of the caloric restriction phase (t1) as well as when the individuals reached the steady state phase at the end of the program (t2), which was usually six to eight months after the starting point (t0). The analysis of that samples revealed a significant reduction in circulating myeloid NK-cells upon weight reduction and maintenance in parallel to improved insulin sensitivity measured by HOMA-IR and reduced systemic inflammation as assessed via circulating concentrations of the high-sensitivity C-reactive protein (hsCRP) (Fig. 3E-H). In line with the observations in mice, obese human subjects display increased levels of CD11b^{hi}IL6Ra⁺ NK-cells which decrease upon weight loss alongside with improved systemic inflammation and insulin sensitivity.

Next, the gene expression signatures of murine and human CD11b^{hi}NK-cells were compared. Therefore, CD11b^{hi}IL6Ra⁺ NK-cells and CD11b⁺IL6Ra⁻ NK-cells were purified by flow cytometry cell sorting (FACS) (gated on viable CD3- cells excluding CD16-CD56- cells) from the circulation of lean and obese human subjects and mRNA deep-sequencing analyses was performed. Unsupervised hierarchical cluster analysis of differentially expressed genes allowed for separation of gene expression signatures between cell types irrespective of body weight. When the lists of genes upregulated in CD11b^{hi} NK-cells in humans and mice were compared, 553 genes were found regulated in both species representing an overlap of 48.6% of the genes found in murine CD11b^{hi} NK-cells. Gene ontology analyses of genes similarly regulated in both species revealed over-representation of myeloid marker genes in human CD11b^{hi}IL6Ra⁺ NK-cells versus CD11b⁺IL6R⁻ NK-cells. Taken together, our analysis confirmed the increased occurrence of a previously undescribed myeloid NK-cell subpopulation in murine and human obesity.

### Example 4: Contribution of Myeloid NK-cells in obesity and insulin resistance in mice

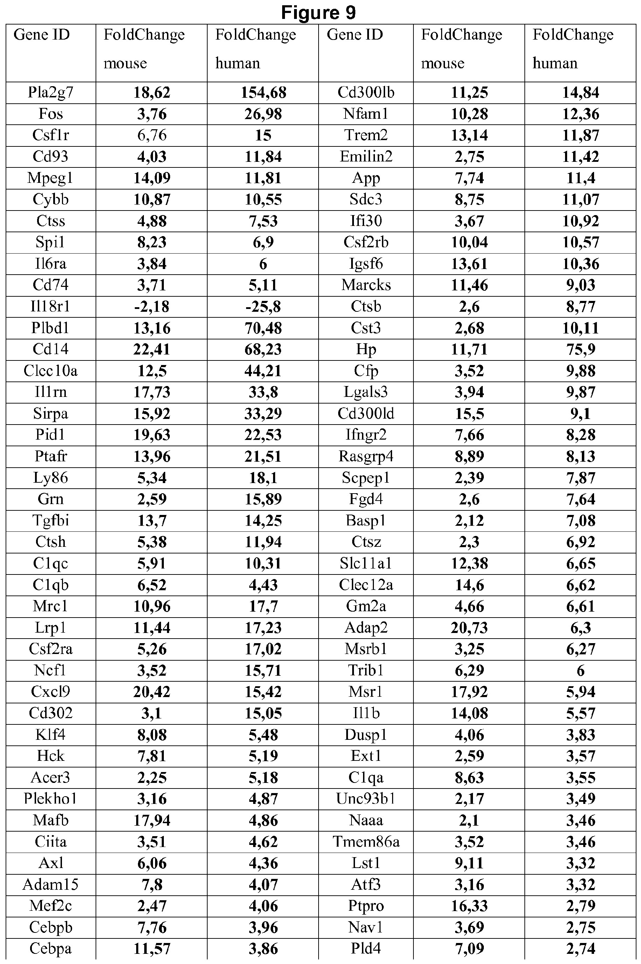

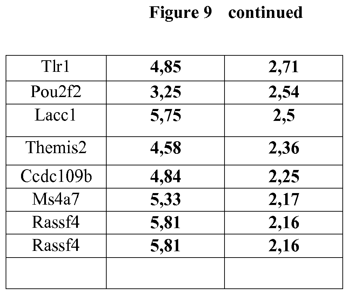

To understand the contribution of the CD11b^{hi} NK-cell population in the development of obesity and/or insulin resistance, the inventors decided to selectively deplete these cells in mice. To this end, they capitalized on the fact, that classical NK-cells - in contrast to myeloid NK-cells - lack expression of the Csf1r gene. By crossing Csf1r^{-loxSTOPlox-DTR}-^{mice} to Ncr1^{Cre}-mice a mouse line was generated, in which NK-cells inducible express the human diphtheria toxin receptor (DTR) only upon Csf1r-gene activation, which enable to specifically deplete Csf1r⁺ NK-cells (i.e. CD11b^{hi} NK-cells) by diphtheria toxin (DT) injections *in vivo* (Fig. 4A). NK-cell specific expression of the Ncr1^{Cre}-transgene was confirmed by flow cytometry in PBMC derived from Ncr1^{Cre}/tdTomato-fluorescent-protein reporter mice (Fig 9A).

To investigate the role of Csf1r⁺Ncr1⁺ (i.e. CD11b^{hi} NK-cells) in obesity, the inventors subjected NK^{Csf1r_DTR} mice and the respective control littermates to HFD-feeding from the age of 6 weeks on (Fig. 4B). At the age of 8 weeks, following two weeks of HFD-feeding and prior to DT-application, neither body weight nor insulin and glucose tolerance differed between NK^{Csf1r_DTR}-mice and floxed controls (Fig. 4C, D). From that time point on, the animals received repetitive intraperitoneal injections of DT (5ng/g body weight) every 5 days (Fig. 4B). Monitoring of body weights during the course of DT-applications revealed that 6 weeks after the beginning of DT-treatment, NK^{Csf1r/DTR}-mice exhibited reduced body weights compared to DT-treated control mice (Fig. 4C). After 9 weeks, the reduction of body weight reached statistical significance and, at the end of the experiment, DT-treated NK^{Csf1r/DTR}-mice weighed 15% less than their DT-treated controls (Fig. 4C). Strikingly, at this point DT-treated NK^{Csf1r/DTR}-mice presented with significantly improved insulin and glucose tolerance (Fig. 4E) and significantly reduced adiposity as assessed by computed tomography (CT) scans (Fig. 4F). Flow cytometry analyses confirmed the successful reduction of CD11b^{hi} NK-cells in the circulation of DT-treated NK^{Csf1r/DTR}-mice albeit the overall number of total or mature NK-cells remained unaltered (Fig. 4G). The improvements in obesity and insulin resistance were accompanied by decreased concentrations of circulating pro-inflammatory cytokines in DT treated NK^{Csf1r/DTR}-mice (Fig. 4H). Collectively, specific, DT-mediated ablation of Csf1r⁺Ncr1⁺DTR, i.e. myeloid NK-cells, reduced obesity, insulin resistance and the systemic pro-inflammatory tone associated with HFD-feeding.

### Example 5: Investigation of the Effect of Abrogation of IL6Ra-signaling from NK-cells of mice

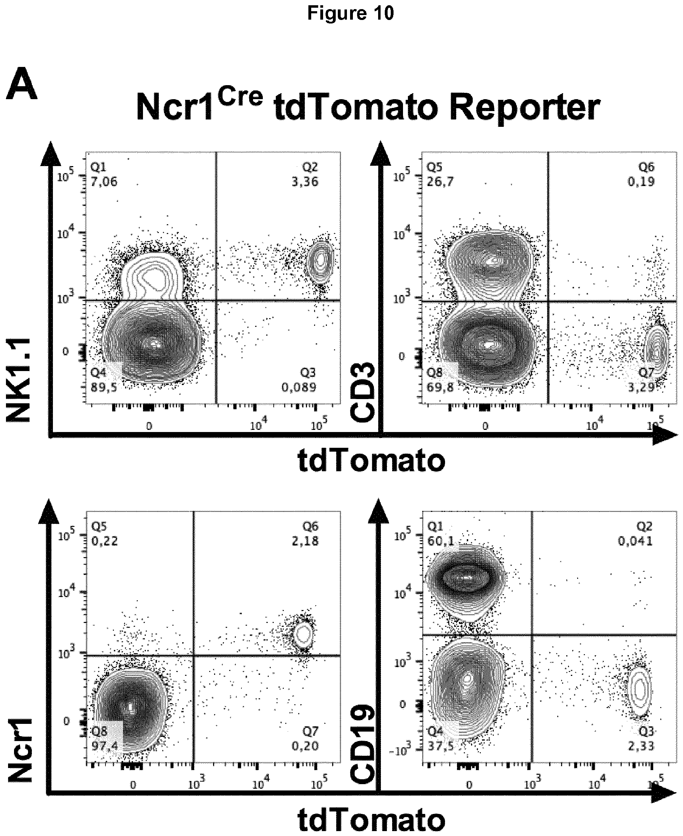

IL6Ra-expression discriminates between mature and myeloid (CD11b^{hi}) NK-cells and IL-6 is consistently increased in circulation of obese mouse models and humans. Thus, the contribution of IL6Ra-signaling in NK-cells during the development of obesity and obesity-associated insulin resistance *in vivo* was investigated. Therefore, mice carrying a loxP-flanked IL6Ra-gene were crossed to Ncr1-Cre mice to specifically delete IL6Ra expression in Ncr1⁺ NK-cells. Such IL6Ra^{ΔNK}-mice and the respective control littermates were then subjected to either CD- or HFD-feeding from the age of 6 weeks on. While body weight remained unaltered between CD-fed IL6Ra^{ΔNK}- and control mice (Fig. 10A), HFD-fed IL6Ra^{ΔNK}-mice exhibited significantly reduced body weights (Fig. 10A), PGAT and liver masses as well as reduced total fat contents (Fig. 10B, C) compared to HFD-fed control mice. Histological examination of PGAT revealed a significant reduction in adipocyte size in IL6Ra^{ΔNK}-mice compared to controls. Reduced body weights in IL6Ra^{ΔNK}-mice upon HFD-feeding resulted from increased energy expenditure as assessed by indirect calorimetry, while food intake of these mice remained unaltered during the night phase and was even slightly increased during the light phase in IL6Ra^{ΔNK}-mice. Consistent with protection from obesity, IL6Ra^{ΔNK}-mice also exhibit reduced hepatic steatosis. Analysis of NK-cells isolated from HFD-fed IL6Ra^{ΔNK}- and control mice confirmed successful and specific disruption of IL6Ra expression in NK-cells. These data led us to conclude that, abrogation of IL6Ra-signaling in NK-cells protects from obesity and hepatic steatosis upon HFD-feeding.

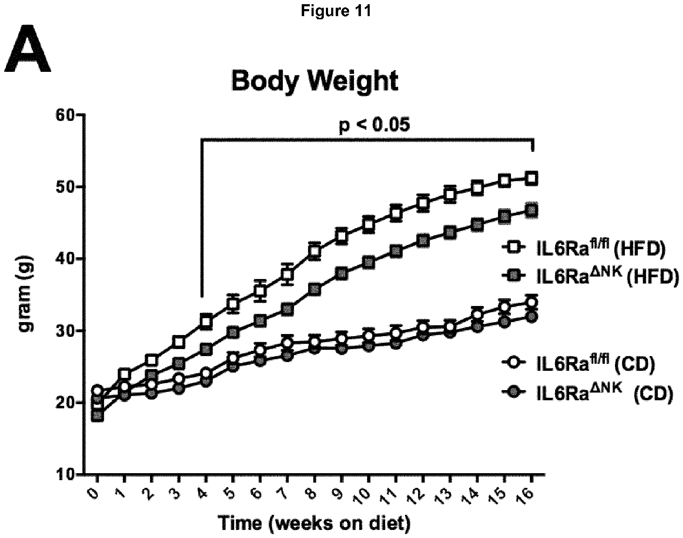

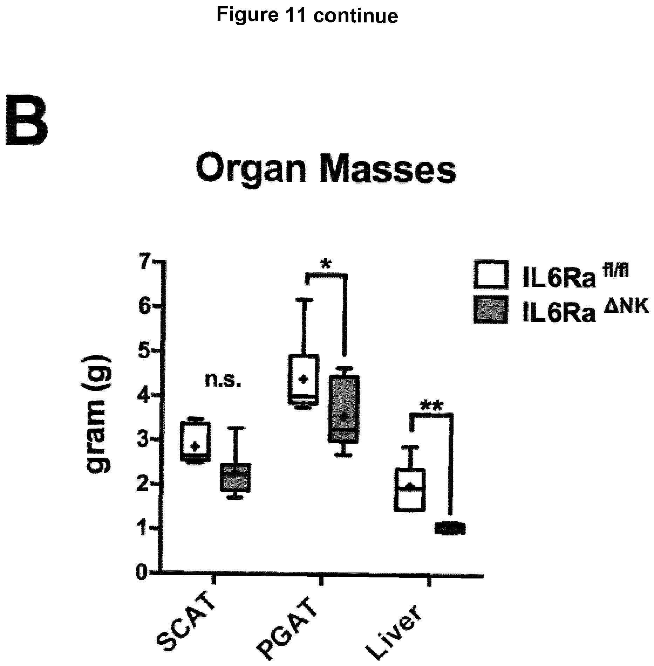

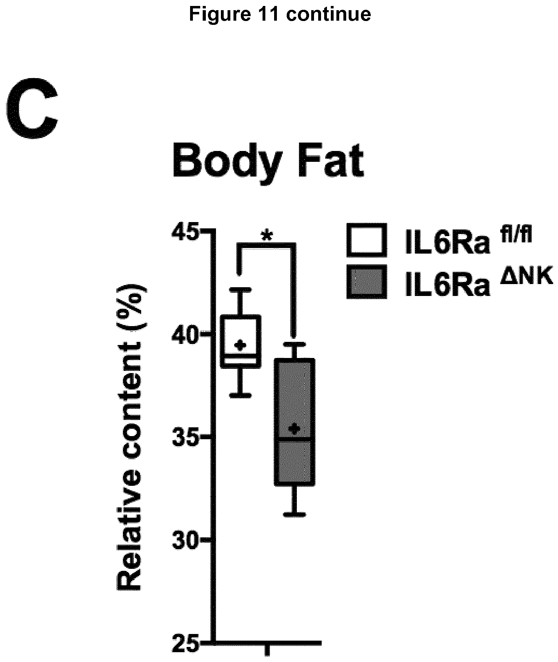

To investigate the consequence of NK-cell specific IL6Ra-deletion for the manifestation of obesity-associated inflammation, the inventors determined total (CD45⁺) immune cell infiltration in liver and PGAT of 22-week old IL6Ra^{ΔNK}- and littermate control mice after 16 weeks of HFD-feeding. This analysis revealed a significant reduction of CD45⁺ immune cells in PGAT of HFD-fed IL6Ra^{ΔNK}-mice compared to controls (Fig. 11A). Similarly, the number of PGAT-infiltrating T cells and NK-cells were reduced in these mice (Fig. 11B, C). In line with the observations during obesity development in wildtype mice, no major differences in NK-cell maturation between IL6Ra^{ΔNK}- and control mice (Fig. 11D) were detected. In contrast, relative numbers of myeloid NK-cells were significantly reduced both in PGAT and circulation of IL6Ra^{ΔNK}-mice (Fig. 5A). In addition, IL6Ra^{ΔNK}-mice exhibited a clear reduction of F4/80 immunoreactive crown-like structures in PGAT compared to controls. Collectively, IL6Ra-inactivation in NK-cells protected from obesity paralleled by a reduction of myeloid NK-cells and obesity-associated macrophage infiltration in PGAT.

To further define the consequences of reduced CD11b^{hi} NK-cell-formation in obese IL6Ra^{ΔNK}-mice, gene expression profiles were analyzed by mRNA deep-sequencing in PGAT and livers of HFD-fed IL6Ra^{ΔNK}- and control mice. The resulting gene expression profiles were subjected to gene set enrichment analyses, which revealed that genes found to be down regulated in livers and PGAT of IL6Ra^{ΔNK}-mice related to immune response, leukocyte migration and activation compared to control mice. Conversely, over represented gene ontologies in genes overexpressed in IL6Ra^{ΔNK}-mice compared to controls clustered in pathways associated with glucose and lipid homeostasis. Taken together, the alterations of immune responses in IL6Ra^{ΔNK}-mice translated into reduced inflammatory gene expression signatures and improved metabolic control in liver and PGAT of these animals.

### Example 6: Determination of insulin resistance in IL6Ra^{ΔNK}-mice during HFD-feeding

To study the consequences of reduced myeloid NK-cell-formation and attenuated obesity-associated inflammation on glucose homeostasis in IL6Ra^{ΔNK}-mice, the inventors performed glucose and insulin tolerance tests in HFD-fed IL6Ra^{ΔNK}-mice and littermate controls. This analysis revealed improved glucose tolerance and insulin sensitivity in IL6Ra^{ΔNK}-mice compared to control animals (Fig. 5B).

To further dissect tissue-specific effects of attenuated IL6Ra-signaling in NK-cells on insulin sensitivity and glucose homeostasis, hyperinsulinemic-euglycemic clamp studies were performed in HFD-fed IL6Ra^{ΔNK}-mice and littermate controls. Here, IL6Ra^{ΔNK}-mice required a significantly higher glucose infusion rate compared to control mice to maintain a similar degree of glycaemia, further underscoring the clearly improved overall insulin sensitivity in these animals (Fig. 5C). Similarly, IL6Ra^{ΔNK}-mice exhibited drastic improvement in insulin's ability to suppress hepatic glucose production and to promote glucose uptake into skeletal muscle and PGAT (Fig. 5C). On a molecular level this was reflected by an increased ability of insulin to stimulate AKT-phosphorylation in skeletal muscle and PGAT of HFD-fed IL6Ra^{ΔNK}-mice. Consistent with an improvement in inflammatory gene expression and insulin action, also obesity-induced JNK-activation was attenuated in liver and PGAT of IL6Ra^{ΔNK}-mice.

### Example 7: Investigation of the effect of Stat3 deletion in NK-cells

Stat3 is a central signaling component downstream of IL-6. Given the importance of IL6Ra-signaling for myeloid NK-cell formation, the inventors therefore conditionally inactivated Stat3 in NK-cells by crossing Ncr1^{Cre}-mice to Stat3^{flox}-mice. Six-week old Stat3^{ΔNK}-mice and their control littermates were subjected to HFD-feeding, and body weights were weekly assessed over a period of 12 weeks. Prior to HFD-feeding, body weights were similar in both 13 genotypes. In contrast, beginning with four weeks of HFD-feeding, Stat3^{ΔNK}-mice gained significantly less weight than the controls, and at the end of the experiment (12 weeks of HFD-feeding) Stat3^{ΔNK}-mice weighed 15% less than the controls (Fig. 6A). Reduced weight gain was reflected in a 44% reduction of fat mass assessed by computed tomography (CT) scans at this time point (Fig. 6B). Histological analyses of organs revealed less PGAT associated crown-like structures and reduced hepatic steatosis in Stat3^{ΔNK}-mice. Strikingly, Stat3^{ΔNK}-mice presented with drastically improved insulin sensitivity and glucose tolerance after 12 weeks of HFD-feeding. Of note, a slightly improved insulin action could be detected prior to HFD (Fig. 6C, D). These metabolic improvements correlated with a significant reduction of myeloid NK-cells in PGAT, whereas - like our previous observations in IL6Ra^{ΔNK}-mice - other NK-cell subpopulations remained unaltered (Fig. 6E).

Thus, the HFD-elicited, IL6Ra-dependent formation of myeloid NK-cells predominantly depends on Stat3-mediated signaling in NK-cells.

## Claims

1. An ex-vivo method for determining myeloid NK-cells comprising the steps of:
a) providing a sample containing myeloid NK-cells;
b) marking the myeloid NK-cells of the sample by means of at least one marking reagent; and
c) detecting the myeloid NK-cells.

2. The method of Claim 1, further comprising a step b') after the step b) b') separating the marked myeloid NK-cells from the sample.

3. The method of Claim 1 or 2, further comprising a step d) after the step c):
d) quantifying the marked myeloid NK-cells.

4. The method of any one of Claims 1 - 3, wherein the myeloid NK-cells are **characterized by** upregulation of at least 50% of the genes selected from the following group consisting of Pla2g7, Fos, Csf1r, Cd93, Mpeg1, Cybb, Ctss, Spi1, Il6ra, Cd74, Plbd1, Cd14, Clec10a, Il1rn, Sirpa, Pid1, Ptafr, Ly86, Grn, Tgfbi, Ctsh, C1qc, C1qb, Mrc1, Lrp1, Csf2ra, Ncf1, Cxcl9, Cd302, Cd300lb, Nfam1, Trem2, Emilin2, App, Sdc3, Ifi30, Csf2rb, Igsf6, Marcks, Ctsb, Cst3, Hp, Cfp, Lgals3, Cd300ld, Ifngr2, Rasgrp4, Scpep1, Fgd4, Basp1, Ctsz, Slc11a1, Clec12a, Gm2a, Adap2, Msrb1, Trib1, Msr1, Il1b, Klf4, Hck, Acer3, Plekho1, Mafb, Ciita, Axl, Adam15, Mef2c, Cebpb, Cebpa, Dusp1, Ext1, C1qa, Unc93b1, Naaa, Tmem86a, Lst1, Atf3, Ptpro, Nav1, Pld4, Tlr1, Pou2f2, Lacc1, Themis2, Ccdc109b, Ms4a7, and Rassf4.

5. Depletion of myeloid NK-cells for use in medicine.

6. Depletion of myeloid NK-cells according to Claim 5 for use in the treatment of obesity, insulin resistance, diabetes, autoimmune diseases, cancer, chronic infections or inflammation.

7. A method for diagnosis of a disease associated with and/or caused by myeloid NK-cells comprising the steps:
a) providing a sample containing myeloid NK-cells;
b) marking the myeloid NK-cells of the sample by means of a marking reagent; and
c) detecting the marked myeloid NK-cells.

8. The method of Claim 7, further comprising steps d) and e) after the step c):
d) quantifying the marked myeloid NK-cells; and
e) comparing the quantified value of the marked myeloid differentiated NK-cells with a standard value.

9. The method of Claim 7 or 8, wherein the disease associated with and/or caused by myeloid NK-cells is selected from a group consisting of obesity, insulin resistance, diabetes, autoimmune diseases, cancer, chronic infections and inflammation.

10. The method of Claim 9, wherein the disease associated with and/or caused by myeloid NK-cells is is obesity-induced diabetes.

11. The method of any one of Claims 7-10, further comprising a step f):
f) detecting and quantifying STAT3 and p-STAT3 of the sample.

12. A method for determining whether a candidate agent reduces or inhibits a myeloid NK-cell population comprising:
a) providing a sample containing the myeloid NK-cell population;
b) contacting the sample containing the myeloid NK-cell population with the candidate agent;
c) marking the myeloid NK-cell population of the sample by means of a marking reagent;
d) detecting the myeloid NK-cell population; and
e) determining if the candidate agent reduces or inhibits the myeloid NK-cell population.

13. The method of Claim 12, wherein the candidate agent is a small organic non-peptidic molecule, a peptidic compound, a nucleic acid, or a metal complex.

14. The method of Claim 12 or 13, wherein step e) reads: e) determining if the candidate agent inhibits activity of myeloid NK-cells.

15. The method of Claim 12, 13 or 14, wherein step e) includes determining if the candidate agent inhibits the activity of at least one protein of a group containing IL6Ra, Csf1r, gp130, JAK1/2, Spi1 and STAT3.
